# EUROPEAN PATENT APPLICATION

(11) **EP 4 424 705 A1**
(43) Date of publication of application: **04.09.2024**
(21) Application number: 22887202.4
(22) Date of filing: 28.10.2022
(51) Int. Cl.: C07K 16/00, C12N 5/10, C12N 7/00, C12N 7/01, C12N 15/13, C12N 15/33, C12N 15/86, C12N 15/864, C12P 21/02, C07K 14/005

(54) **COMPOSITION FOR CELL ADDITION**

(30) Priority: 29.10.2021 JP 2021178303; 26.04.2022 JP 2022072677
(71) Applicant: AGC INC., Chiyoda-ku, Tokyo 1008405 (JP)
(72) Inventor: SHIINA, Shunsuke, Tokyo 100-8405 (JP); HASEGAWA, Junichi, Tokyo 100-8405 (JP); KUBODERA, Kiyomi, Tokyo 100-8405 (JP)
(74) Representative: Müller-Boré & Partner Patentanwälte PartG mbB
(86) International application number: PCT/JP2022/040520
(87) International publication number: WO 2023/074877

(57) **Abstract**

The present invention aims to provide, in the production of proteins such as antibody, a method for improving the production amount of the protein; a method for improving the genome productivity and/or the integrity of the viral genome in the production of viral vectors; and compositions therefor. The production of at least one desired component in a cell can be enhanced by contacting the cell with a composition containing an organic solvent.

## Description

### [Technical Field]

The present invention relates to production methods of components desired in cells, particularly proteins such as antibody and the like, and viral vector; methods for introducing nucleic acids encoding components desired in cells, particularly proteins such as antibody and the like, and viral vector; methods for enhancing the expression of nucleic acids encoding desired components, particularly proteins such as antibody and the like, and viral vector, in cells into which the nucleic acids have been introduced; and compositions and the like therefor.

### [Background Art]

Biopharmaceutical products are pharmaceutical products that are manufactured using the ability of microorganisms and cells to produce proteins (hormones, enzymes, antibodies, etc.) by applying genetic recombination technology, cell culture technology, and the like, and are becoming the mainstream in medicaments in recent years. A gene for a protein of interest, such as an antibody, is introduced into a cell (preferably mammalian cell), and the cell produces the protein of interest.

The methods of gene transfer are mainly divided into two: methods using retrovirus, lentivirus, and adeno-associated viral vector, and physicochemical methods such as lipofection method, electroporation method, and microinjection method.

On the other hand, in the medical fields such as gene therapy and the like, as a method for introducing a gene into the cells of mammals inclusive of human, methods using virus-derived gene transfer vectors (hereinafter viral vectors) are generally used. Viral vectors are vectors that enable transfer of desired genes, etc. into the target, by modifying naturally occurring viruses using genetic recombination technology, and technological development has been progressing in recent years.

As viruses from which viral vectors are derived, viruses having an envelope such as retrovirus, lentivirus, Sendai virus, herpes virus, and the like, and viruses not having an envelope (hereinafter non-enveloped virus) such as adenovirus, adeno-associated virus (hereinafter AAV), and the like are known.

In particular, AAV is seen as a promising vector for gene transfer used in gene therapy methods since it can infect cells of a wide variety of species including humans, can also infect non-dividing cells that have completed differentiation, and is not pathogenic to humans and thus poses little concern about side effects, and virus particles are physicochemically stable.

Generally, AAV is produced by introducing plasmid DNA necessary for AAV production into mammalian cells such as human embryonic kidney cells 293 (HEK293 cells) and the like, and is known to have quality issues such as the packaging rate of the AAV genome within the AAV capsid and fragmentation of the AAV genome packaged within the capsid, in addition to low productivity.

Non-enveloped viral vectors such as adenovirus, AAV, and the like exhibit broad tissue affinity and cell affinity, capacity for large expression cassettes, and high transduction efficiency, and have characteristics desirable for gene delivery.

For the development and commercial production of biopharmaceutical products and viral vector drugs, it is important to transfect cells with high efficiency and increase the amount of target products produced within cells. In order to increase transfection efficiency and gene transcription activity, addition of enhancers is known (Patent Literature 1, Non Patent Literatures 1 and 2).

However, methods for producing a viral vector with improved viral genome production amount and viral genome integrity, and compositions therefor are not known.

### [Citation List]

### [Patent Literature]

[Patent Literature 1]
JP 2020-524498 A

### [Non Patent Literature]

[Non Patent Literature 1]
   Huang, Xinping et al., J Virol Methods. 2013 Nov; 193(2):270-7.
[Non Patent Literature 2]
   Cervera, Laura et al., Appl Microbiol Biotechnol. 2015 Dec; 99 (23) :9935-49.

### [Summary of Invention]

### [Technical Problem]

The present invention aims to provide a method for improving the production amount of proteins such as antibody and the like; a method for improving the genome productivity of viral vector and/or the integrity of the viral genome in the production of viral vectors; and compositions therefor.

### [Solution to Problem]

In producing proteins such as antibody and the like, it is necessary to prepare protein-producing cells. Preparation of protein-producing cells generally requires introduction of plasmid DNA involved in protein production, including protein-encoding DNA, into the cell and enhancement of expression of the introduced plasmid DNA within the cell.

In producing viral vectors, the virus from which viral vectors are derived can be prepared, for example, in the case of AAV, by introducing plasmid DNA necessary for AAV production into mammalian cells such as HEK293 cell and the like, and similar to protein production, introduction of plasmid DNA necessary for AAV production into the cells and enhanced expression of the introduced plasmid DNA in the cells are necessary.

In view of the above-mentioned problem, the present inventors have intensively studied the conditions for transfecting plasmids into cells and the conditions for enhancing the expression of plasmids in cells. As a result, it was found that the viral genome production amount and genome integrity of the viral vector were improved by transfecting in the presence of organic solvents such as alcohols and dimethyl sulfoxide (DMSO), as compared with those in the absence thereof. In the case of protein-producing cells, the production amount of protein was found to have further improved.

In addition, it was also found that, in the case of protein-producing cells, the amount of protein produced is improved, and in the case of viral vectors, the amount of viral genome produced and the genome integrity of the viral vector are improved, not only during transfection, but also by bringing cells into contact with organic solvents such as alcohols and dimethyl sulfoxide (DMSO).

Furthermore, preferred reaction conditions therefor were successfully determined, whereby the present invention was completed.

Accordingly, the present invention provides the following.
[1] A composition for addition to cells, which comprises an organic solvent, wherein the organic solvent enhances in the cell the production of at least one desired component.
[2] The composition of the above-mentioned [1], wherein a signal related to cell survival has been regulated in the cell after the addition compared to the cell before the addition.
[3] The composition of the above-mentioned [2], wherein the regulation of the signal is enhancement of the signal.
[4] The composition of the above-mentioned [3], wherein the signal is a protein kinase A signal.
[5] The composition of the above-mentioned [3] or [4], wherein the regulation of the signal is by regulation of expression of a factor related to the signal.
[6] The composition of the above-mentioned [5], wherein the regulation of the expression of the factor is enhancement.
[7] The composition of the above-mentioned [5], wherein the regulation of the expression of the factor is suppression.
[8] The composition of the above-mentioned [2], wherein the regulation of the signal is the suppression of the signal.
[9] The composition of the above-mentioned [8], wherein the signal is at least one kind selected from the group consisting of a necroptosis signal and an interferon signal.
[10] The composition of the above-mentioned [8] or [9], wherein the suppression of the signal is by the regulation of the expression of the factor related to the signal.
[11] The composition of the above-mentioned [10], wherein the regulation of the expression of the factor is enhancement.
[12] The composition of the above-mentioned [10], wherein the regulation of the expression of the factor is suppression.
[13] The composition of any of the above-mentioned [1] to [12], which increases survival of the cell and/or activity of the cell.
[14] The composition of any of the above-mentioned [1] to [13], wherein the desired component is an antibody and/or a viral vector.
[15] The composition of any of the above-mentioned [1] to [14], which is for the transfection of a nucleic acid.
[16] The composition of any of the above-mentioned [1] to [14], which is for the enhanced expression of a nucleic acid.
[17] The composition of the above-mentioned [15] or [16], wherein the nucleic acid is a DNA encoding a protein.
[18] The composition of the above-mentioned [17], wherein the aforementioned DNA is a DNA involved in the production of at least one kind selected from the group consisting of an antibody and a viral vector.
[19] The composition of the above-mentioned [18], wherein the aforementioned viral vector is derived from a virus belonging to the Parvoviridae or Retroviridae.
[20] The composition of the above-mentioned [18] or [19], wherein the aforementioned viral vector is an adeno-associated virus (AAV) vector.
[21] The composition of any of the above-mentioned [17] to [20], wherein the aforementioned DNA is one or a plurality of DNAs encoding a viral packaging protein and a helper protein.
[22] The composition of the above-mentioned [21], wherein the virus package protein is at least one kind selected from the group consisting of REP and CAP.
[23] The composition of the above-mentioned [21], wherein the helper protein is at least one kind selected from the group consisting of E1A, E1B, E2A, E4, and VA derived from adenovirus.
[24] The composition of any of the above-mentioned [1] to [23], wherein the organic solvent comprises an alcohol and/or dimethyl sulfoxide.
[25] A method for enhancing the production of at least one desired component in a cell, comprising contacting the cell with a composition comprising an organic solvent.
[26] A method for enhancing the production of at least one desired component in a cell, comprising incubating a reaction mixture comprising the cell and an organic solvent.
[27] The method of the above-mentioned [25] or [26], wherein the aforementioned cell is a cell into which a DNA involved in the production of at least one desired component has been introduced.
[28] The method of the above-mentioned [25] or [26], wherein a signal related to cell survival has been regulated in the cell after contact with the composition comprising the organic solvent, or after incubation, compared to the cell before the contact or incubation.
[29] The method of the above-mentioned [28], wherein the regulation of the signal is enhancement of the signal.
[30] The method of the above-mentioned [29], wherein the signal is a protein kinase A signal.
[31] The method of the above-mentioned [29] or [30], wherein the signal enhancement is by the regulation of the expression of the factor related to the signal.
[32] The method of the above-mentioned [31], wherein the regulation of the expression of the factor is enhancement.
[33] The method of the above-mentioned [31], wherein the regulation of the expression of the factor is suppression.
[34] The method of the above-mentioned [28], wherein the regulation of the signal is the suppression of the signal.
[35] The method of the above-mentioned [34], wherein the signal is at least one kind selected from the group consisting of a necroptosis signal and an interferon signal.
[36] The method of the above-mentioned [34] or [35], wherein the suppression of the signal is by the regulation of the expression of the factor related to the signal.
[37] The method of the above-mentioned [36], wherein the regulation of the expression of the factor is enhancement.
[38] The method of the above-mentioned [36], wherein the regulation of the expression of the factor is suppression.
[39] The method of any of the above-mentioned [25] to [38], which increases survival of the cell and/or activity of the cell.
[40] The method of any of the above-mentioned [25] to [39], wherein the desired component is an antibody and/or a viral vector.
[41] A method for transfecting a nucleic acid encoding a desired component into a cell, comprising incubating a reaction mixture comprising a nucleic acid encoding at least one desired component, a cell, and an organic solvent.
[42] The method of the above-mentioned [41], which enhances the expression of the transfected nucleic acid.
[43] The method of the above-mentioned [41] or [42], wherein a signal related to cell survival has been regulated in the cell after the transfection compared to the cell before the transfection.
[44] The method of the above-mentioned [43], wherein the regulation of the signal is enhancement of the signal.
[45] The method of the above-mentioned [44], wherein the signal is a protein kinase A signal.
[46] The method of the above-mentioned [44] or [45], wherein the regulation of the signal is by regulation of expression of a factor related to the signal.
[47] The method of the above-mentioned [46], wherein the regulation of the expression of the factor is enhancement.
[48] The method of the above-mentioned [46], wherein the regulation of the expression of the factor is suppression.
[49] The method of the above-mentioned [43], wherein the regulation of the signal is the suppression of the signal.
[50] The method of the above-mentioned [49], wherein the signal is at least one kind selected from the group consisting of a necroptosis signal and an interferon signal.
[51] The method of the above-mentioned [49] or [50], wherein the suppression of the signal is by the regulation of the expression of the factor related to the signal.
[52] The method of the above-mentioned [51], wherein the regulation of the expression of the factor is enhancement.
[53] The method of the above-mentioned [51], wherein the regulation of the expression of the factor is suppression.
[54] The method of any of the above-mentioned [41] to [53], wherein the nucleic acid is a DNA encoding a protein.
[55] The method of the above-mentioned [54], wherein the aforementioned DNA is a DNA involved in the production of at least one kind selected from the group consisting of an antibody and a viral vector.
[56] The method of the above-mentioned [55], wherein the aforementioned viral vector is derived from a virus belonging to the Parvoviridae or Retroviridae.
[57] The method of the above-mentioned [55] or [56], wherein the aforementioned viral vector is an adeno-associated virus (AAV) vector.
[58] The method of any of the above-mentioned [54] to [57], wherein the aforementioned DNA is one or a plurality of DNAs encoding a viral packaging protein and a helper protein.
[59] The method of the above-mentioned [58], wherein the virus package protein is at least one kind selected from the group consisting of REP and CAP.
[60] The method of the above-mentioned [58], wherein the helper protein is at least one kind selected from the group consisting of E1A, E1B, E2A, E4, and VA derived from adenovirus.
[61] The method of any of the above-mentioned [41] to [60], wherein the organic solvent comprises an alcohol and/or dimethyl sulfoxide.
[62] The method of the above-mentioned [61], wherein a content of the organic solvent is not more than 2 V/V% based on the total amount of the reaction mixture.
[63] The method of any of the above-mentioned [41] to [62], wherein the reaction mixture further comprises a transfection reagent.
[64] The composition of the above-mentioned [24] or the method of any of the above-mentioned [61] to [63], wherein the alcohol is at least one kind selected from the group consisting of methanol, ethanol, isopropanol, and glycerol.
[65] A method for transfecting a DNA encoding a desired component into a cell, comprising
   a first step of incubating a cell suspension comprising a cell and a DNA encoding at least one desired component,
   a second step of preparing a reaction mixture by adding an organic solvent to the incubated cell suspension, and
   a third step of incubating the reaction mixture, wherein
   the organic solvent is an alcohol, and
   a content of the alcohol is not more than 2 V/V% based on the total amount of the aforementioned reaction mixture.
[66] A method for producing a transfected cell, comprising transfecting a cell by the method of any of the above-mentioned [41] to [64].
[67] A method for producing a viral vector, comprising obtaining a viral vector from a cell obtained by the production method of the above-mentioned [66].

### [Advantageous Effects of Invention]

According to the method of the present invention, it is possible to produce proteins by cells more superior in production amount, and to produce viral vectors by cells more superior in viral genome production amount and/or genome integrity.

### [Brief Description of Drawings]

[Fig. 1A]

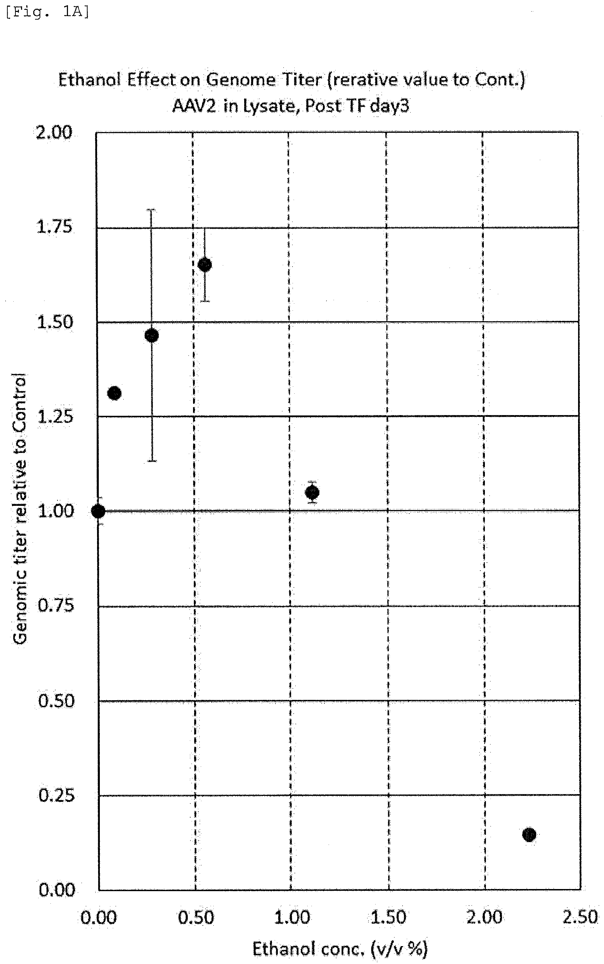

   Fig. 1A is a graph showing the investigation results of the effect of ethanol addition on intracellular AAV2 production during AAV2 production.
[Fig. 1B]

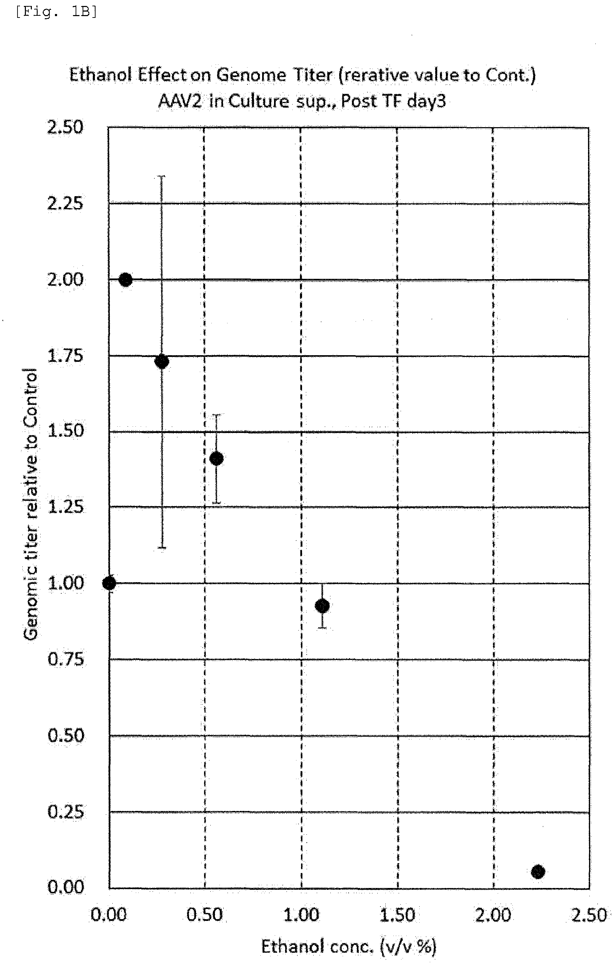

   Fig. 1B is a graph showing the investigation results of the effect of ethanol addition on AAV2 production amount in cell culture supernatant during AAV2 production.
[Fig. 1C]

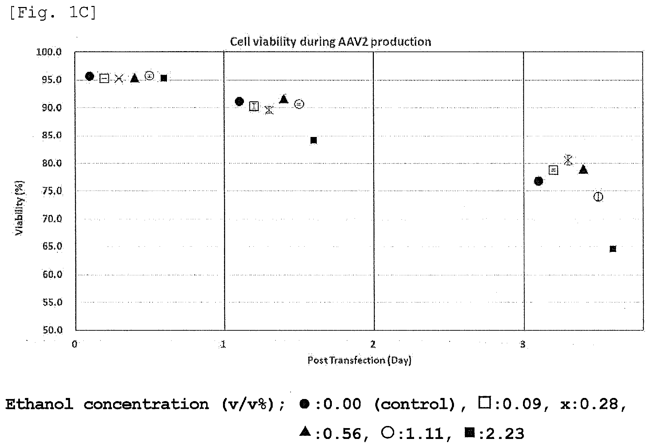

   Fig. 1C is a graph showing the investigation results of the effect of ethanol addition on the improvement of cell survival rate during AAV2 production.
[Fig. 2A]

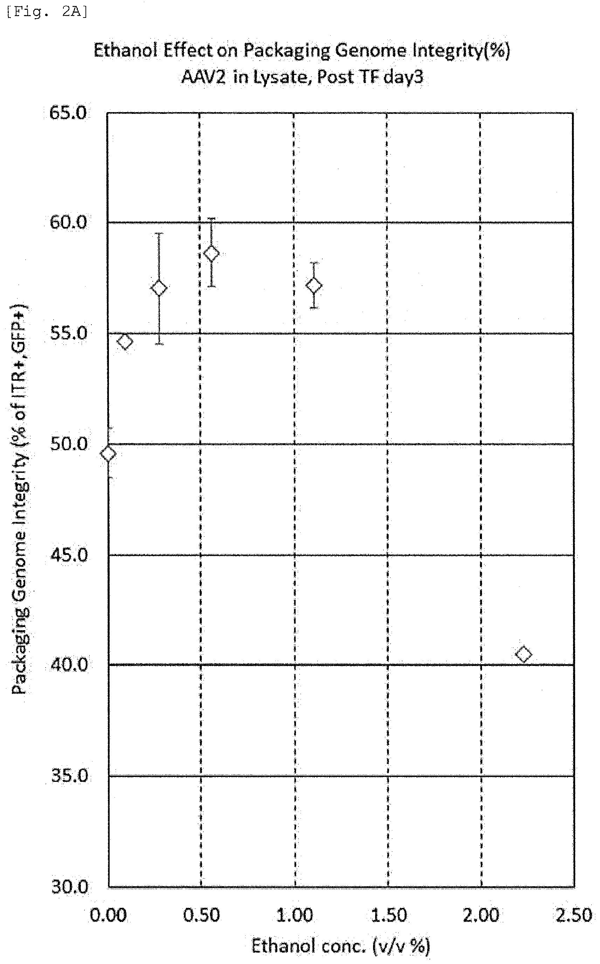

   Fig. 2A is a graph showing the investigation results of the effect of ethanol addition on the integrity of genome in intracellular AAV2 during AAV2 production.
[Fig. 2B]

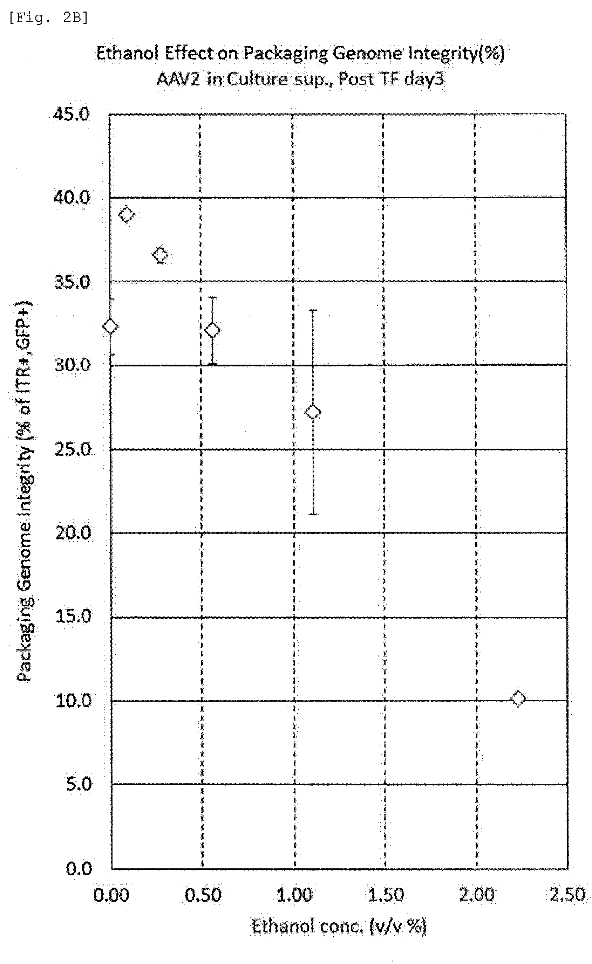

   Fig. 2B is a graph showing the investigation results of the effect of ethanol addition on the integrity of genome in AAV2 in cell culture supernatant during AAV2 production.
[Fig. 3]

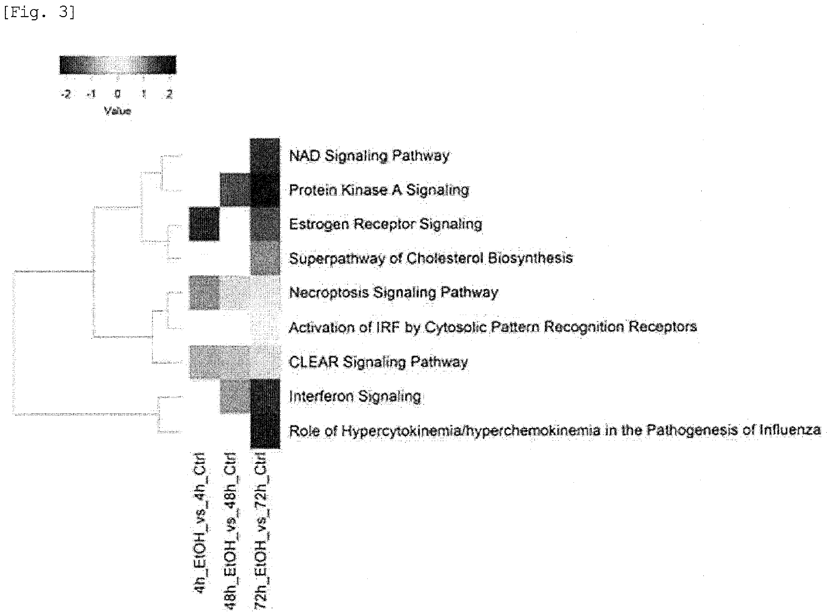

   Fig. 3 is a heat map diagram of pathways activated or suppressed upon addition of ethanol. Time shows the time after transfection of plasmid DNA for AAV2 production.
[Fig. 4]

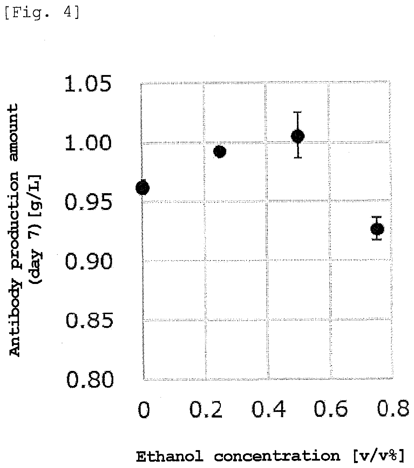

   Fig. 4 is a graph showing the investigation results of the effect of ethanol addition on the production amount of antibody in cells derived from Chinese hamster.

### [Description of Embodiments]

The present invention is described below. Unless otherwise specified, the terms used in the present specification have meanings generally used in the pertinent field.

The present invention provides a composition for addition to cells, which contains an organic solvent, wherein the organic solvent enhances in the cell the production of at least one desired component, particularly, a composition for increasing survival of the cell, a composition for enhancing the expression of nucleic acids (for example, nucleic acids (DNA) involved in the production of proteins such as antibodies) in cells (hereinafter to be also referred to as "the composition for enhancing nucleic acid expression of the present invention" in the case of use for enhancing expression of nucleic acids), or a composition used when transfecting nucleic acids (DNA) involved in the production of proteins such as antibody or viral vectors (hereinafter to be also referred to as "the composition for transfection of the present invention" in the case of use for transfection) into cells (hereinafter these are also collectively referred to as "the composition of the present invention"). The composition of the present invention preferably contains an alcohol and/or dimethyl sulfoxide (DMSO) as an organic solvent.

In the present invention, the composition for enhancing nucleic acid expression characteristically enhances the expression of nucleic acids within cells. For example, by adding the composition for enhancing nucleic acid expression of the present invention to a cell engineered to consistently express a nucleic acid (DNA) involved in the production of proteins such as antibody and the like, the expression of the nucleic acid can be enhanced, and the production of a desired component (e.g., proteins such as antibody and the like) can be enhanced.

In the present invention, the composition for transfection is characteristically used when transfecting nucleic acids into cells, but it is not a necessary component (so-called transfection reagent) for introducing nucleic acids into cells. When used, it contributes to the improvement of the production amount of proteins such as antibody and the like and viral vectors produced within cells, and the integrity of the produced viral genome.

In the present invention, to "enhance the production of a desired component" means significantly increasing the production amount of a desired component in cells compared to cells before treating with the composition of the present invention. The desired component is a component to be obtained, and includes, for example, components of protein or nucleic acid such as protein (e.g., antibody), virus vector and the like. When the desired component is a viral vector, it also intends to include improving the integrity of the genome packaged within the cell.

In the present invention, to "enhance survival of the cell" means significantly improving the survival ability even when placed under a stress (load), for example, compared to cells before treatment with the composition of the present invention. Examples of the stress include detachment and reseeding of cells (so-called passage), introduction of nucleic acids into cells (so-called transfection), and intracellular production of proteins and viruses derived from the introduced nucleic acids.

In the cells treated with the composition of the present invention, signals related to cell survival may be regulated and are preferably regulated as compared with the state before being treated with the composition of the present invention (prior to addition). Such cells are hereinafter also to be referred to as "the cell of the present invention".

The present invention provides a method for enhancing the production of at least one desired component in a cell, including contacting the cell with a composition containing an organic solvent.

One embodiment of the method is a method of transfecting a nucleic acid encoding at least one desired component whose production is required to be enhanced into a cell in the presence of an organic solvent (hereinafter also to be referred to as "the transfection method of the present invention").

Another embodiment of the method is a method of incubating a reaction mixture containing cells into which a nucleic acid encoding at least one desired component whose production is required to be enhanced has been introduced, and an organic solvent.

One embodiment of the method includes a step of incubating a reaction mixture containing a nucleic acid encoding at least one desired component, cells and an organic solvent, where the organic solvent is preferably alcohol and/or dimethyl sulfoxide.

Another embodiment of the method includes a step of incubating a reaction mixture containing cells into which a nucleic acid encoding at least one desired component has been introduced and an organic solvent, where the organic solvent is preferably alcohol and/or dimethyl sulfoxide.

One embodiment of the method includes
a first step of incubating a cell suspension comprising a cell and a DNA encoding at least one desired component,
a second step of preparing a reaction mixture by adding an organic solvent to the incubated cell suspension, and
a third step of incubating the reaction mixture, wherein
the organic solvent is an alcohol, and the content of the alcohol is not more than 2 V/V% based on the total amount of the aforementioned reaction mixture.

The amount of the organic solvent to be used in the reaction mixture of the present invention is not particularly limited as long as the above-mentioned effects are achieved. In order to bring cells into contact with a composition containing an organic solvent, cells and an organic solvent are essential. For example, when the organic solvent is an alcohol (e.g. ethanol), the organic solvent is generally not more than 2 V/V% based on the total amount of the reaction mixture containing these essential components. If it is not more than the above-mentioned upper limit, the production of at least one desired component can be enhanced without killing the cell, and, for example, the amount of viral genome produced within the cell and/or the integrity of the viral genome can be improved. Moreover, the production amount of proteins (e.g., antibody) within cells can be improved without killing the cells. Preferably, the content of the alcohol to be used is not more than 1.9 V/V%, not more than 1.8 V/V%, not more than 1.7 V/V%, not more than 1.6 V/V%, not more than 1.5 V/V%, not more than 1.4 V/V%, not more than 1.3 V/V%, not more than 1.2 V/V%, not more than 1.1 V/V%, not more than 1 V/V%, not more than 0.9 V/V%, not more than 0.8 V/V%, not more than 0.7 V/V%, or not more than 0.6 V/V%, based on the total amount of the aforementioned reaction mixture. The lower limit of the amount of the organic solvent to be used in the total amount of the reaction mixture is not particularly limited as long as the above-mentioned effects are achieved, and is a concentration exceeding 0 V/V%. Preferably, the content of the alcohol is not less than 0.05 V/V%, not less than 0.06 V/V%, not less than 0.07 V/V%, not less than 0.08 V/V%, not less than 0.09 V/V%, not less than 0.1 V/V%, not less than 0.11 V/V%, not less than 0.12 V/V%, not less than 0.13 V/V%, not less than 0.14 V/V%, not less than 0.15 V/V%, not less than 0.16 V/V%, not less than 0.17 V/V%, not less than 0.18 V/V%, not less than 0.19 V/V%, or not less than 0.2 V/V%, based on the total amount of the aforementioned reaction mixture. If it is not less than the above-mentioned lower limit, production of at least one desired component can be enhanced. For example, the amount to be used can be increased or decreased as appropriate according to the kind of the organic solvent.

The amount of the organic solvent to be used in the reaction mixture of the present invention can enhance the production of at least one desired component produced within the cell, and is preferably 0.05 to 2 V/V%, more preferably 0.1 to 1.5 V/V%, further preferably 0.2 to 1 V/V%, particularly preferably 0.2 to 0.8 V/V%, most preferably 0.2 to 0.56 V/V%, in order to improve (enhance), for example, the production amount of protein (e.g., antibody) and/or the production amount of viral genome.

The amount of the organic solvent to be used in the reaction mixture of the present invention during transfection is not particularly limited as long as the above-mentioned effects are achieved. For transfection of nucleic acids into cells, nucleic acids, cells, and an organic solvent are essential. For example, when the organic solvent is an alcohol (e.g. ethanol), the organic solvent is generally not more than 2 V/V% based on the total amount of the reaction mixture containing these essential components. If it is not more than the above-mentioned upper limit, by using during transfection of nucleic acids into cells, the production of at least one desired component can be enhanced without killing the cell, and, for example, the amount of viral genome produced within the cell and/or the integrity of the viral genome can be improved. Moreover, the production amount of proteins (e.g., antibody) within cells can be improved without killing the cells. Preferably, the content of the alcohol to be used is not more than 1.9 V/V%, not more than 1.8 V/V%, not more than 1.7 V/V%, not more than 1.6 V/V%, not more than 1.5 V/V%, not more than 1.4 V/V%, not more than 1.3 V/V%, not more than 1.2 V/V%, not more than 1.1 V/V%, not more than 1 V/V%, not more than 0.9 V/V%, not more than 0.8 V/V%, not more than 0.7 V/V%, or not more than 0.6 V/V%, based on the total amount of the aforementioned reaction mixture. The lower limit of the amount of the organic solvent to be used in the total amount of the reaction mixture is not particularly limited as long as the above-mentioned effects are achieved, and is a concentration exceeding 0 V/V%. Preferably, the content of the alcohol is not less than 0.05 V/V%, not less than 0.06 V/V%, not less than 0.07 V/V%, not less than 0.08 V/V%, not less than 0.09 V/V%, not less than 0.1 V/V%, not less than 0.11 V/V%, not less than 0.12 V/V%, not less than 0.13 V/V%, not less than 0.14 V/V%, not less than 0.15 V/V%, not less than 0.16 V/V%, not less than 0.17 V/V%, not less than 0.18 V/V%, not less than 0.19 V/V%, or not less than 0.2 V/V%, based on the total amount of the aforementioned reaction mixture. If it is not less than the above-mentioned lower limit, by using during transfection of nucleic acids into cells, production of at least one desired component can be enhanced. For example, the amount to be used can be increased or decreased as appropriate according to the kind of the organic solvent.

The amount of the organic solvent to be used in the reaction mixture of the present invention during transfection can enhance the production of at least one desired component produced within the cell, and is preferably 0.05 to 2 V/V%, more preferably 0.1 to 1.5 V/V%, further preferably 0.2 to 1 V/V%, particularly preferably 0.2 to 0.8 V/V%, most preferably 0.2 to 0.56 V/V%, in order to improve (enhance), for example, the production amount of protein (e.g., antibody) and/or the production amount of viral genome.

When the desired component is a viral vector, a preferred embodiment of the amount of the organic solvent to be used in the reaction mixture during transfection is 0.05 to 2 V/V%, 0.1 to 1.8 V/V%, 0.2 to 1.5 V/V%, or 0.28 to 1.11 V/V%, in order to improve (enhance) integrity of the viral genome to be produced within the cell.

The present invention provides a method for producing a transfected cell, including transfecting a plasmid expressing a desired component into a cell by the transfection method of the present invention. Furthermore, the present invention provides a method for producing a viral vector, including obtaining the viral vector from a transfected cell.

### 1. Virus vector (virus)

In the present invention, the viruses from which the viral vector to be produced is derived include, but are not limited to, viruses having an envelope such as retroviruses, lentiviruses, Sendai virus, herpes viruses, and the like, viruses free of an envelope (hereinafter non-enveloped virus) such as adenoviruses, AAV, and the like, and the like. The envelope is formed when the virus buds by penetrating membranes such as the nucleus, endoplasmic reticulum, Golgi apparatus, plasma membrane, and cell membrane, generally accompanies host-derived proteins or viral proteins expressed on the host cell membrane, and plays an important role in infecting target cells.

Specifically, DNA viruses such as adenoviruses, parvovirus, papovaviruses, human papillomaviruses, and the like, and RNA viruses such as rotaviruses, coxsackieviruses, enteroviruses, sapoviruses, noroviruses, polioviruses, echoviruses, type A hepatitis virus, type E hepatitis virus, rhinoviruses, astroviruses, and the like can be mentioned. Retroviridae virus, Adenoviridae virus, and Parvoviridae virus are more preferred, and Parvoviridae adeno-associated virus (AAV) is particularly preferred. AAV has a regular icosahedral outer shell (capsid) without an envelope and a linear single-stranded DNA inside the shell. The capsid has three capsid proteins (VP1, VP2, and VP3). In the present specification, AAV includes wild-type virus and derivatives thereof, and includes all serotypes and clades unless otherwise specified. While there are various reports on AAV serotypes, at least 15 serotypes of AAV1, AAV2, AAV3a, AAV3b, AAV4, AAV5, AAV6, AAV7, AAV8, AAV9, AAV10, AAVrh.10, AAV11, AAV12, and AAV13 are known as the serotypes of AAV that infect humans.

In the present invention, the "virus" includes, in addition to natural viruses, recombinant virus particles that have been modified based on natural viruses to remove pathogenicity and include a region for introducing foreign genes. Such recombinant virus particles are also to be referred to as viral vectors. In the present invention, natural viruses and recombinant virus particles (viral vectors) are sometimes collectively referred to as viruses.

Viral vectors can be used for genetic manipulation (i.e., "cloning vectors") for the introduction/transfer of nucleic acids into cells and for the transcription or translation of nucleic acids inserted into cells. An "expression vector" is a specialized vector that contains a gene or nucleic acid sequence with regulatory regions necessary for expression in a host cell. Vector nucleic acid sequences generally include at least an origin of replication for proliferation propagation in a cell and, optionally, additional elements such as heterologous polynucleotide sequence, expression control elements (e.g., promoter, enhancer), introns, ITR (could be plural), selection markers (e.g., antibiotic resistance), and polyadenylated signals.

Viral vectors are derived from or based on one or more nucleic acid elements that contain viral genome.

In the present invention, the "viral vector" means a particle constituted of a shell of a capsid protein. Furthermore, in the present invention, the "viral vector" means not only those containing a viral genome (nucleic acid form), but also hollow particles that are virus-like particles constituted only of capsid proteins not containing a viral genome. It is preferable to obtain a "viral vector" containing a viral genome (in the form of a nucleic acid) inside a capsid protein shell.

### 2. Transfection

Methods for producing viral vectors are broadly classified into methods that use viruses and methods that do not use viruses. In the present invention, the method includes transfecting a nucleic acid encoding a desired component, preferably a nucleic acid encoding one or more desired components, into cells.

Specifically, one or more DNAs encoding a viral packaging protein and a helper protein are introduced into cells by using a transfection reagent. A DNA encoding a viral packaging protein and a DNA encoding a helper protein may be provided in separate plasmids, but they can also be provided in one plasmid. When a nucleic acid other than DNA involved in viral vector production is introduced, a vector plasmid containing a nucleic acid containing a target gene sandwiched between ITRs (Inverted terminal repeats) at both ends is preferably transfected simultaneously with one or more DNAs encoding a viral packaging protein and a helper protein. Examples of the target gene include functional nucleic acid, mRNA or fragments thereof, combinations thereof, and the like, recited in the "3. Nucleic acid" described below.

A "transfection reagent" is any compound and/or composition that increases the uptake of one or more nucleic acids into one or more target cells, and is not particularly limited. Various transfection reagents are known to those of ordinary skill in the art. As preferred transfection reagent, one or more compounds and/or compositions including cationic polymers such as polyethyleneimine (PEI) and the like, positively charged amino acid polymers such as polylysine, polyarginine, and the like, positively charged dendrimers, fractures dendrimers, cationic β-cyclodextrin-containing polymers (CD polymers), DEAE-dextran, and the like can be mentioned.

The term "viral packaging protein", in the case of AAV, for example, refers to non-AAV-derived viral and/or cellular functions upon which AAV is dependent for its replication. In the case of the viral packaging protein used in the present invention, for example, AAV, it is "AAV packaging protein", and has AAV-derived sequences that function in trans for productive AAV replication. AAV packaging protein is encoded by the major AAV open reading frame (ORF), REP, and CAP. REP proteins have been shown to have many functions, including, among others, recognition, binding, and nicking of AAV origin of DNA replication; DNA helicase activity; and regulation of transcription from AAV (or other heterologous) promoters. CAP (capsid) proteins provide the necessary packaging functions.

The term "nucleic acid encoding helper protein" generally refers to a nucleic acid molecule (could be plural) that includes a nucleotide sequence encoding a protein that provides helper function (could be plural). A vector containing nucleic acid (could be plural) encoding helper protein (could be plural) can be transfected into a suitable host cell, where the vector can be used for viral (e.g., AAV) particle production in the host cell.

Helper proteins include, for example, adenovirus-derived elements, such as E1A, E1B, E2, E4, VA, and the like.

In one embodiment, the AAV vector is produced by transfecting a vector plasmid in which the region between ITRs is replaced with the target gene, a packaging plasmid expressing REP and CAP, and a helper plasmid expressing E2A, E4, and VA, together into 293 cells (expressing E1A and E1B, described below).

The vector "genome" refers to a recombinant nucleic acid sequence that is ultimately packaged or encapsulated to form a viral (e.g., AAV) particle. When a recombinant vector is constructed or produced using a recombinant plasmid, the vector genome does not contain any portion of the "plasmid" that does not correspond to the vector genome sequence of the recombinant plasmid. This non-vector genomic portion of the recombinant plasmid is referred to as the "plasmid backbone". While the portion is important for cloning and amplification of plasmid, which is a step necessary for propagation and production of recombinant virus, it is not itself packaged or encapsulated into viral (e.g., AAV) particles. Therefore, the vector "genome" refers to the nucleic acid that is packaged or encapsulated by a virus (e.g., AAV).

### 3. Nucleic acid

The term "nucleic acid" can also be referred to as a polynucleotide, and is used interchangeably in the present specification to refer to all forms of nucleic acids and polynucleotides, including deoxyribonucleic acid (DNA) and ribonucleic acid (RNA). Nucleic acids and polynucleotides include genomic DNA, cDNA and antisense DNA, as well as spliced or unspliced mRNA, rRNA, tRNA and inhibitory DNA or RNA (RNAi, for example, small or short hairpin (sh) RNA, microRNA (miRNA), small or short interfering (si) RNA, trans-splicing RNA, or antisense RNA). Nucleic acids and polynucleotides include naturally occurring, synthetic, and intentionally modified or altered sequences.

The nucleic acid is preferably a DNA encoding a protein. When the composition for transfection of the present invention is used for the production of viral vector, the DNA includes at least DNA involved in the production of the viral vector. Examples of the protein encoded by the DNA include viral packaging protein and helper protein. The nucleic acid to be transfected into cells may contain functional nucleic acid, mRNA or fragments thereof, or a combination thereof, in addition to the DNA involved in the production of the viral vector. The "functional nucleic acid" refers to a specific biological function, such as enzymatic function, catalytic function, or biological inhibition or promotion function (e.g., inhibition or promotion of transcription, translation) in a living body or in a cell, preferably in a cell. Specifically, for example, RNA interference agent, nucleic acid aptamer (including RNA aptamer and DNA aptamer), antisense DNA, ribozyme (including deoxyribozyme), U1 adapter, molecular beacon, riboswitch, transcription factor binding region, and the like can be mentioned. Two or more types of functional nucleic acids may be encapsulated. mRNA is an mRNA that encodes any protein, and may be either mRNA precursor or mature mRNA.

Genes encoding therapeutic proteins (e.g., antibody-based drug, Fc fusion protein, anticoagulant, blood factor, bone morphogenetic protein, engineered protein scaffold, enzyme, growth factor, hormone, interferon, interleukin, cytokine, thrombolytic agents, and any combination thereof), drug resistance genes (e.g., kanamycin resistance gene, streptomycin resistance gene, spectinomycin resistance gene, gentamicin resistance gene, chloramphenicol resistance gene, erythromycin resistance gene), genes encoding labeled proteins (e.g., fluorescent protein, luminescent protein), and the like may be included.

### 4. Composition

The composition of the present invention is a composition for addition to cells, which contains an organic solvent, wherein the organic solvent enhances in the cell the production of at least one desired component. The "desired component" includes, for example, the "viral vector" described in the above-mentioned section 1., the "nucleic acid" described in the section 3. and proteins encoded thereby, and the like. It is preferably a protein and/or a viral vector. By treating with the composition of the present invention, the survival of the cell can be increased. The composition of the present invention can be used by contacting with cells and can enhance the expression of desired components within cells. In addition, the composition of the present invention can be used in transfecting nucleic acids into cells. By using the composition of the present invention when transfecting a nucleic acid into a cell when the nucleic acid is, for example, DNA involved in the production of proteins, the production amount of the protein (e.g., antibody) produced within the cell can be increased. When the nucleic acid is, for example, DNA involved in the production of viral vector, the amount of viral genome produced within cells and/or the integrity of the viral genome are/is improved. Preferred compositions are those that improve (increase) the amount of protein produced, and improve (enhance) the amount of viral genome produced and the integrity of the viral genome.

In addition, when the composition to be used in the present invention is used in transfecting a nucleic acid into cells, the production amount of a viral vector containing the viral genome inside the capsid protein shell and/or the integrity of the viral genome contained in the viral vector can be improved (enhanced). A preferred composition is one that improves (enhances) the production amount of a viral vector containing the viral genome inside the capsid protein shell and the integrity of the viral genome contained in the viral vector.

Furthermore, when the composition to be used in the present invention is used in transfecting a nucleic acid into cells, the production amount of a viral vector containing an integrated viral genome is improved.

As the organic solvent, alcohols and dimethyl sulfoxide (DMSO) can be mentioned, and alcohols are preferred.

Alcohol is substance in which the hydrogen atom of hydrocarbon is replaced with a hydroxyl group. In the present specification, they are collectively referred to as alcohols. The alcohol is preferably a lower alcohol having 1 to 6 carbon atoms. For example, it is at least one selected from the group consisting of methanol, ethanol, isopropanol, and glycerin.

The amount of the organic solvent to be used in the composition of the present invention is not particularly limited as long as theabove-mentioned effects are achieved. In order to bring cells into contact with a composition containing an organic solvent, cells and an organic solvent are essential. For example, when the organic solvent is an alcohol (e.g. ethanol), the organic solvent is generally used at not more than 2 V/V% based on the total amount of the reaction mixture containing these essential components. If it is not more than the above-mentioned upper limit, the production of at least one desired component can be enhanced without killing the cell, and, for example, the amount of viral genome produced within the cell and/or the integrity of the viral genome can be improved. Moreover, the production amount of proteins (e.g., antibody) within cells can be improved without killing the cells. Preferably, the content of the alcohol to be used is not more than 1.9 V/V%, not more than 1.8 V/V%, not more than 1.7 V/V%, not more than 1.6 V/V%, not more than 1.5 V/V%, not more than 1.4 V/V%, not more than 1.3 V/V%, not more than 1.2 V/V%, not more than 1.1 V/V%, not more than 1 V/V%, not more than 0.9 V/V%, not more than 0.8 V/V%, not more than 0.7 V/V%, or not more than 0.6 V/V%, based on the total amount of the reaction mixture. The lower limit of the amount of the organic solvent to be used in the total amount of the reaction mixture is not particularly limited as long as the above-mentioned effects are achieved, and is a concentration exceeding 0 V/V%. Preferably, the content of the alcohol to be used is not less than 0.05 V/V%, not less than 0.06 V/V%, not less than 0.07 V/V%, not less than 0.08 V/V%, not less than 0.09 V/V%, not less than 0.1 V/V%, not less than 0.11 V/V%, not less than 0.12 V/V%, not less than 0.13 V/V%, not less than 0.14 V/V%, not less than 0.15 V/V%, not less than 0.16 V/V%, not less than 0.17 V/V%, not less than 0.18 V/V%, not less than 0.19 V/V%, or not less than 0.2 V/V%, based on the total amount of the aforementioned reaction mixture. If it is not less than the above-mentioned lower limit, production of at least one desired component can be enhanced. For example, the amount to be used can be increased or decreased as appropriate according to the kind of the organic solvent.

The amount of the organic solvent to be used in the composition of the present invention can enhance the production of at least one desired component produced within the cell, and is preferably 0.05 to 2 V/V%, more preferably 0.1 to 1.5 V/V%, further preferably 0.2 to 1 V/V%, particularly preferably 0.2 to 0.8 V/V%, most preferably 0.2 to 0.56 V/V%, based on the total amount of the reaction mixture, in order to improve (enhance), for example, the production amount of protein (e.g., antibody) and/or the production amount of viral genome.

The amount of the organic solvent to be used in the composition of the present invention during transfection is not particularly limited as long as the above-mentioned effects are achieved. For transfection of nucleic acids into cells, nucleic acids, cells, and an organic solvent are essential. For example, when the organic solvent is an alcohol (e.g. ethanol), the organic solvent is generally used at not more than 2 V/V% based on the total amount of the reaction mixture containing these essential components. If it is not more than the above-mentioned upper limit, by using during transfection of nucleic acids into cells, the production of at least one desired component can be enhanced without killing the cell, and, for example, the amount of viral genome produced within the cell and/or the integrity of the viral genome can be improved. Moreover, the production amount of proteins (e.g., antibody) within cells can be improved without killing the cells. Preferably, the content of the alcohol to be used is not more than 1.9 V/V%, not more than 1.8 V/V%, not more than 1.7 V/V%, not more than 1.6 V/V%, not more than 1.5 V/V%, not more than 1.4 V/V%, not more than 1.3 V/V%, not more than 1.2 V/V%, not more than 1.1 V/V%, not more than 1 V/V%, not more than 0.9 V/V%, not more than 0.8 V/V%, not more than 0.7 V/V%, or not more than 0.6 V/V%, based on the total amount of the reaction mixture during transfection. The lower limit of the amount of the organic solvent to be used in the total amount of the reaction mixture is not particularly limited as long as the above-mentioned effects are achieved, and is a concentration exceeding 0 V/V%. Preferably, the content of the alcohol to be used is not less than 0.05 V/V%, not less than 0.06 V/V%, not less than 0.07 V/V%, not less than 0.08 V/V%, not less than 0.09 V/V%, not less than 0.1 V/V%, not less than 0.11 V/V%, not less than 0.12 V/V%, not less than 0.13 V/V%, not less than 0.14 V/V%, not less than 0.15 V/V%, not less than 0.16 V/V%, not less than 0.17 V/V%, not less than 0.18 V/V%, not less than 0.19 V/V%, or not less than 0.2 V/V%, based on the total amount of the aforementioned reaction mixture. If it is not less than the above-mentioned lower limit, by using during transfection of nucleic acids into cells, production of at least one desired component can be enhanced. For example, the amount to be used can be increased or decreased as appropriate according to the kind of the organic solvent.

The amount of the organic solvent to be used in the reaction mixture of the present invention during transfection can enhance the production of at least one desired component produced within the cell, and is preferably 0.05 to 2 V/V%, more preferably 0.1 to 1.5 V/V%, further preferably 0.2 to 1 V/V%, particularly preferably 0.2 to 0.8 V/V%, most preferably 0.2 to 0.56 V/V%, based on the total amount of the reaction mixture during transfection, in order to improve (enhance), for example, the production amount of protein (e.g., antibody) and/or the production amount of viral genome.

When the desired component is a viral vector, a preferred embodiment of the amount of the organic solvent to be used in the composition during transfection is 0.05 to 2 V/V%, 0.1 to 1.8 V/V%, 0.2 to 1.5 V/V%, or 0.28 to 1.11 V/V%, in order to improve (enhance) integrity of the viral genome to be produced within the cell.

### 5. Reaction mixture for transfection

The reaction mixture for transfection in the present invention is not particularly limited as long as it contributes to improving the production amount of proteins such as antibody and viral vectors produced in cells and/or the integrity of the viral genome, and is a composition containing a cell, a nucleic acid, and an organic solvent. More preferred reaction mixture for transfection is a composition containing a cell, a nucleic acid, an organic solvent, and a transfection reagent.

As a method for preparing the reaction mixture for transfection in the present invention, a cell, a nucleic acid, and an organic solvent may be added simultaneously, or an organic solvent may be added after preparing a cell suspension containing cells and nucleic acids. Furthermore, when the reaction mixture for transfection in the present invention contains a transfection reagent, a cell, a nucleic acid, an organic solvent, and a transfection reagent may be added simultaneously, or a cell suspension containing a cell, a nucleic acid, and a transfection reagent may be prepared and thereafter an organic solvent may be added.

By using the reaction mixture for transfection of the present invention during transfection, the survival rate of cells is improved.

In addition, the reaction mixture for transfection of the present invention may contain known components (other components) that significantly act on transfection, in addition to the aforementioned components. Other components include valproic acid, sodium salt thereof, sodium butyrate, caffeine, and the like. When other components are contained, cells, nucleic acids, organic solvents, and other components, and transfection reagents when desired, may be added at the same time, or an organic solvent and other components may be added after preparing a cell suspension containing cells, nucleic acids, and transfection reagents when desired.

### 6. Cells and production of transfected cells

In the present invention, a cell used to produce at least one desired component (e.g., protein, viral vector) is not particularly limited as long as the desired component can be produced in the inside thereof and can proliferate. When the desired component is a viral vector, it is preferably a cell into which a part of the gene required for the production of viral vector has been introduced, and is a packaging cell that does not produce virus by itself. Examples include one derived from eukaryotic cells and preferably HEK293 cells, HEK293T cell, HEK293F cells, HEK293FT cells, G3T-hi cells, Sf9 cells, commercially available cell lines for virus production, AAV293 cells, and the like, which have high transfection efficiency. Human-derived cells are more preferred and HEK293 cells are particularly preferred. In addition, for example, the aforementioned HEK293 cells and the like constitutively express adenovirus E1 protein. Such cells that are modified to transiently or constitutively express one or some of the proteins required for recombinant adenovirus may also be used.

Examples of the element required for the production of recombinant adenovirus include (A) REP protein, CAP protein, (B) adenovirus-derived elements, for example, E1A, E1B, E2, E4, VA genes, and the like. The form of these nucleic acids is not limited, and they can be introduced into cells to be used as one or more nucleic acid constructs that can supply each element in the cells and are loaded into plasmids or viral vectors. When these elements are not expressed in cells, one or more of (A) and/(B) is/are transfected into the cell as a nucleic acid encoding a desired component, as described above.

When the desired component is a protein such as an antibody, the cell of the present invention may be a cell that originally produces the protein, or may be a modified cell into which a gene necessary for producing the protein has been introduced. The method for introducing a gene necessary for producing the protein varies depending on the type of the desired protein and is selected as appropriate. When the protein is an antibody, generally, a method including cloning the genes for the heavy chain and light chain of an immunoglobulin molecule, and introducing same into host cells (preferably mammalian cells) by using a vector with high expression efficiency can be mentioned. The variable regions (Fab region or ScFV region) of heavy chain and light chain genes may be cloned and reconstituted in vitro. As the protein-producing cells, established cultured cells are preferred. For example, when an antibody is produced, monoclonal antibody-producing hybridoma, established B cell, and the like can be exemplified. Examples of cells that can be modified by introducing genes necessary for production of proteins include mammalian cells such as CHO cell, COS cell, HeLa cell, Vero cell, and the like.

In the cells of the present invention, signals related to cell survival are preferably regulated as compared with the state before being treated with the composition of the present invention (prior to addition). For example, when stress (load) is applied to the cells, it is a signal that up-regulates or down-regulates the viability of the cells. Cell survival can be increased by enhancing the signal within the cell in the case of a signal that upregulates cell viability, and by suppressing the signal within the cell in the case of a signal that downregulates cell viability.

In the cells of the present invention, moreover, factors related to signals that upregulate cell viability are preferably regulated as compared with the state before being treated with the composition of the present invention (prior to addition). As used herein, "regulation" refers to the state where, for example, when stress (load) is applied to cells, factors related to the signals that upregulate cell viability are enhanced or suppressed in the cells.

Furthermore, in the cells of the present invention, moreover, factors related to signals that downregulate cell viability are preferably regulated as compared with the state before being treated with the composition of the present invention (prior to addition). As used herein, "regulation" refers to the state where, for example, when stress (load) is applied to cells, factors related to the signals that downregulate cell viability are enhanced or suppressed in the cells.

Each signal and factor varies depending on the type of cell and the stress applied to the cell. Examples include respective signals and factors listed in Table 1 and Table 2.

**[Table 1-1]**

| Pathway name (regulation mode) | effect | factor | regulation mode |
|---|---|---|---|
| NAD Signaling Pathway (enhancement) | cell survival | PRKAG2 | enhancement |
| | growth control | PIK3CB | enhancement |
| | growth control | PIK3C3 | enhancement |
| | | NMNAT1 | enhancement |
| | | DXO | enhancement |
| | | INS | enhancement |
| | chromatin structuring, transcriptional control | H1-6 | enhancement |
| | | NADSYN1 | enhancement |
| | | POLR2I | suppression |
| | | TP53 | suppression |
| | chromatin structuring, transcriptional control | H1-6 | suppression |
| | chromatin structuring, transcriptional control | H1-4 | suppression |
| | | POLR2K | suppression |
| | | PARP9 | suppression |
| | | PIK3CB | suppression |
| | | SLC29A1 | suppression |
| Protein Kinase A Signaling (enhancement) | cytogenesis, growth control | SMAD3 | enhancement |
| | cell survival | PRKAG2 | enhancement |
| | cytogenesis, growth control | NGFR | enhancement |
| | | GNG2 | enhancement |
| | intracellular transport | KDELR1 | enhancement |
| | cytogenesis, growth control | PTPN3 | enhancement |
| | cytogenesis, growth control | PTPN18 | enhancement |
| | | PRKD3 | enhancement |

**[Table 1-2]**

| | | | |
|---|---|---|---|
| | | PRKACG | enhancement |
| | | CAMK4 | enhancement |
| | chromatin structuring, transcriptional control | H1-6 | enhancement |
| | | REL | enhancement |
| | | PLCD3 | enhancement |
| | | PPP1R11 | enhancement |
| | chromatin structuring, transcriptional control | H1-0 | enhancement |
| | cytogenesis, growth control | SMAD4 | enhancement |
| | cell survival | PDE | enhancement |
| | cell survival | LIPE | enhancement |
| | cell survival | PHK | enhancement |
| | cell survival | Glycogen phosohorylase | enhancement |
| | cell survival | Glucose-1-P | enhancement |
| | cell survival | TH | enhancement |
| | cell survival | L-DOPA | enhancement |
| | cell survival | TnI | enhancement |
| | transcription activity, Virus replication | CREB1 | enhancement |
| | transcription activity, Virus replication | EBI3 | enhancement |
| | transcription activity, Virus replication | CTNNB1 | enhancement |
| | transcription activity, Virus replication | Histone H3 | enhancement |
| | transcription activity, Virus replication | CREBBP | enhancement |
| | transcription activity, Virus replication | CREM | enhancement |
| | transcription activity, Virus replication | CREB | enhancement |

**[Table 1-3]**

| | | | |
|---|---|---|---|
| | transcription activity, Virus replication | ATF1 | enhancement |
| | chromatin structuring, transcriptional control | H1-2 | suppression |
| | | PTPRG | suppression |
| | | RAF1 | suppression |
| | | PPP1R14B | suppression |
| | | PRKAR1A | suppression |
| | | PPP1R14C | suppression |
| | chromatin structuring, transcriptional control | H1-6 | suppression |
| | | DUSP9 | suppression |
| | | PXN | suppression |
| | chromatin structuring, transcriptional control | H1-4 | suppression |
| | calcium control, intracellular transduction | ITPR3 | suppression |
| | | GNA13 | suppression |
| | | ANAPC10 | suppression |
| | | ADCY9 | suppression |
| | | AKAP10 | suppression |
| | | TCF3 | suppression |
| | cytogenesis, growth control | PTPRK | suppression |
| | intracellular transduction control | NTN1 | suppression |
| | cell survival | cAMP | suppression |
| | cell survival | BAD | suppression |
| | cell survival | PLN | suppression |
| | cell survival | GSK3 | suppression |
| | cell survival | Glycogen synthase | suppression |
| | transcription activity, Virus replication | NFAT | suppression |
| | transcription activity, Virus replication | ELK1 | suppression |

**[Table 1-4]**

| | | | |
|---|---|---|---|
| Estrogen Receptor Signaling (enhancement) | cell survival | PRKAG2 | enhancement |
| | mitochondrial electron transport system | MT-ND4 | enhancement |
| | external signal cell response | GNG2 | enhancement |
| | | MED20 | enhancement |
| | | MMP15 | enhancement |
| | mitochondrial electron transport system | MT-CYB | enhancement |
| | | REL | enhancement |
| | | PRKACG | enhancement |
| | | RRAS | suppression |
| | | PRKAR1A | suppression |
| | | NDUFB7 | suppression |
| | | SP1 | suppression |
| | | NDUFA3 | suppression |
| | | MED24 | suppression |
| | | ADCY9 | suppression |
| | | RAF1 | suppression |
| | mitochondrial electron transport system | MT-ND4 | suppression |
| | transcriptional control | MED6 | suppression |
| Superpathway of Cholesterol Biosynthesis (enhancement) | cholesterol biosynthesis | SC5D | enhancement |
| | | TM7SF2 | enhancement |
| | | FDFT1 | enhancement |
| | | GGPS1 | enhancement |
| | | DHCR24 | enhancement |
| | lipid metabolism | HADHB | enhancement |
| | lipid metabolism | HADHA | enhancement |
| | | EBP | enhancement |
| | | LBR | enhancement |
| | | ACAT1 | suppression |
| | | MVD | suppression |

**[Table 1-5]**

| | | | |
|---|---|---|---|
| | | NSDHL | suppression |
| | | ACAA2 | suppression |
| | | HMGCS1 | suppression |
| | | MVK | suppression |
| | cholesterol biosynthesis catalyst | TM7SF2 | suppression |
| | cholesterol biosynthesis catalyst | DHCR24 | suppression |
| | cholesterol biosynthesis | MSMO1 | suppression |

| CLEAR Signaling Pathway (enhancement) | | | |
|---|---|---|---|
| Activation of IRF by Cytosilic Pattern Recognition Receptor (enhancement) | cell death induction | FADD | enhancement |
| | | NFKBIE | enhancement |
| | | TBK1 | enhancement |
| | | MAVS | enhancement |
| | | IKBKG | enhancement |
| | | REL | enhancement |
| | apoptosis, necroptosis | RIPK1 | enhancement |
| | | IRF3 | enhancement |
| | | PIN1 | suppression |
| | | JUN | suppression |
| | | DDX58 | suppression |
| | antiviral immune activity | ISG15 | suppression |
| | antiviral immune activity, apoptosis induction | IFIT2 | suppression |
| | immune response, cell death control | CHUK | suppression |
| | extracellular signal transduction | MAPK12 | suppression |

**[Table 2-1]**

| Pathway name (regulation mode) | effect | factor | regulation mode |
|---|---|---|---|
| Necroptosis Signaling Pathway (suppression) | cell activity control, glucose uptake | NGFR | enhancement |
| | intracellular signal transduction | CAPN5 | enhancement |
| | cell survival, differentiation | MERTK | enhancement |
| | cell death induction | FADD | enhancement |
| | immune control, intracellular protein transport | FKBP1A | enhancement |
| | intramitochondrial uptake | TOMM6 | enhancement |
| | apoptosis induction control | TRADD | enhancement |
| | | TSPO | enhancement |
| | | GLUD1 | enhancement |
| | | RBCK1 | enhancement |
| | intramitochondrial transport | TIMM10 | enhancement |
| | | TNFRSF10B | enhancement |
| | apoptosis, necroptosis | RIPK1 | enhancement |
| | cell death induction | MIR512-3p | enhancement |
| | cell death induction | MIR874 | enhancement |
| | cell death induction | MLKL | enhancement |
| | cell death induction | MIR128-1 | enhancement |
| | cell death induction | MIR155 | enhancement |
| | intramitochondrial transport | TIMM44 | suppression |
| | | EIF2AK2 | suppression |
| | intramitochondrial transport | TIMM13 | suppression |
| | immunotransduction system | JAK1 | suppression |
| | | TP53 | suppression |
| | intramitochondrial uptake | TOMM20 | suppression |

**[Table 2-2]**

| | | | |
|---|---|---|---|
| | intramitochondrial transport | TIMM8B | suppression |
| | cell death induction | GLUL | suppression |
| | intramitochondrial transport | TIMM10 | suppression |
| | autoimmunity | TNIP1 | suppression |
| | apoptosis induction control | TRADD | suppression |
| | immune response, cell death control | CHUK | suppression |
| | intramitochondrial uptake | TOMM22 | suppression |
| | | RB1 | suppression |
| | intramitochondrial uptake | TOMM7 | suppression |
| | cell death induction | BIRC2/3 | suppression |
| | cell death induction | CYLD | suppression |
| | cell death induction | FADD | suppression |
| | cell death induction | CFLIPS | suppression |
| | cell death induction | CASP8 | suppression |
| | cell death induction | MIR873 | suppression |
| Interferon Signaling (suppression) | immunity, inflammatory cell proliferation suppression | IFI35 | enhancement |
| | viral proliferation suppression | IFITM3 | enhancement |
| | transcription activation | MED14 | enhancement |
| | apoptosis activation | BAX | enhancement |
| | antiviral immune activity | BCL2 | enhancement |
| | antiviral immune activity | RELA | suppression |
| | immunotransduction system | JAK1 | suppression |
| | antiviral immune activity | IFIT1 | suppression |
| | antiviral immune activity | IFIT3 | suppression |
| | antivirus activation | ISG15 | suppression |
| | antiviral immune activity | STAT1 | suppression |
| | antiviral immune activity | STAT2 | suppression |
| | antiviral immune activity | IPF1 | suppression |
| | antiviral immune activity | IPF9 | suppression |
| | antiviral immune activity | PSMB8 | suppression |
| | antiviral immune activity | IFI6 | suppression |

**[Table 2-3]**

| | | | |
|---|---|---|---|
| | antiviral immune activity | MX1 | suppression |
| | antiviral immune activity | IFITM1 | suppression |
| | antiviral immune activity | IFITM2 | suppression |
| | antiviral immune activity | TAP1 | suppression |
| | antiviral immune activity | BAK1 | suppression |
| Role of Hypercytokinemia/ hyperchemokinemia in the Pathogenesis of influenza (suppression) | antiviral immunity | IRF3 | enhancement |
| | immune response regulation | NFKB1 | enhancement |
| | virus RNA degradation, virus replication inhibition | OAS3 | suppression |
| | virus RNA degradation, virus replication inhibition | OAS2 | suppression |
| | inflammatory chemokine, virus control | CCL5 | suppression |
| | antiviral immune activity | IFIT3 | suppression |
| | antivirus activation | ISG15 | suppression |
| | antiviral immune activity | IFIT2 | suppression |

Signals to be regulated as compared with the state before being treated with the composition of the present invention (prior to addition) include, for example,
NAD signaling (NAD Signaling Pathway) involved in intracellular signal transduction,
protein kinase A signaling (Protein Kinase A Signaling), estrogen receptor signaling involved in gene expression control (Estrogen Receptor Signaling),
cholesterol biosynthesis signaling involved in cell morphology and intracellular maintenance (Superpathway of Cholesterol Biosynthesis),
CLEAR signaling (CLEAR Signaling Pathway),
IRF activating signaling by cytosolic pattern recognition receptor (Activation of IRF by Cytosilic Pattern Recognition Receptor),
necroptosis signaling relating to cell death and immunity against viral infection (Necroptosis Signaling Pathway), interferon signaling (Interferon Signaling), and
signaling involved in hypercytokinemia/hyperchemokinemia (Role of Hypercytokinemia/hyperchemokinemia in the Pathogenesis of influenza), in which NAD signaling, protein kinase A signaling, estrogen receptor signaling, cholesterol biosynthesis signaling, CLEAR signaling, and IRF activating signaling by cytosolic pattern recognition receptor are respectively enhanced, and necroptosis signaling, interferon signaling, and signaling involved in hypercytokinemia/hyperchemokinemia are suppressed.

Preferably, as an enhanced signal, protein kinase A signaling can be mentioned, and as a suppressed signal, necroptosis signaling and interferon signaling can be mentioned.

Factors involved in protein kinase A signaling that, when enhanced, enhance protein kinase A signaling include, for example, SMAD3, PRKAG2, NGFR, GNG2, KDELR1, PTPN3, PTPN18, PRKD3, PRKACG, CAMK4, H1-6, REL, PLCD3, PPP1R11, H1-0, SMAD4, PDE, LIPE, PHK, Glycogen phosohorylase, Glucose-1-P, TH, L-DOPA, TnI, CREB1, EBI3, CTNNB1, Histone H3, CREBBP, CREM, CREB, and ATF1. Factors involved in protein kinase A signaling that, when suppressed, enhance protein kinase A signaling include, for example, H1-2, PTPRG, PAF1, PPP1R14B, PRKAR1A, PPP1R14C, H1-6, DUSP9, PXN, H1-4, ITPR3, GNA13, ANAPC10, ADCY9, AKAP10, TCF3, PTPRK, NTN1, cAMP, BAD, PLN, GSK3, Glycogen synthase, NFAT, and ELK1.

Factors involved in necroptosis signaling that, when enhanced, suppress necroptosis signaling include, for example, NGFR, CAPN5, MERTK, FADD, FKBP1A, TOMM6, TRADD, TSPO, GLUD1, RBCK1, TIMM10, TNFRSF10B, RIPK1, MIR512-3p, MIR874, MLKL, MIR128-1, and MIR155. Factors involved in necroptosis signaling that, when suppressed, suppress necroptosis signaling include, for example, TIMM44, EIF2AK2, TIMM13, JAK1, TP53, TOMM20, TIMM8B, GLUL, TIMM10, TNIP1, TRADD, CHUK, TOMM22, RB1, TOMM7, BIRC2/3, CYLD, FADD, CFLIPS, CASP8, and MIR873.

Factors involved in interferon signaling that, when enhanced, suppress interferon signaling include, for example, IFI35, IFITM3, MED14, BAX, and BCL2. Factors involved in interferon signaling that, when suppressed, suppress interferon signaling include, for example, RELA, JAK1, IFIT1, IFIT3, ISG15, STAT1, STAT2, IPF1, IPF9, PSMB8, IFI6, MX1, IFITM1, IFITM2, TAP1, and BAK1.

If the expression of signals related to cell survival and factors related to signals can be regulated within cells and survival of the cell can be increased, the same effects as when using the composition of the present invention can also be obtained without using the composition of the present invention.

The culturing of cells in the present invention includes a step of transfecting a nucleic acid into cells and a step of producing antibody, protein, and viral vector within the transfected cells, and can be performed under known culture conditions. The culture form of the cells may be suspension culture or adhesive culture. Examples include, but are not limited to, culturing at temperature 30 to 37°C, humidity 95%RH, CO₂ concentration 5 to 10% (v/v). Temperature, humidity, and CO₂ concentration outside the aforementioned ranges may be used as long as the proliferation of cells that produce the desired components and the production of antibodies and virus particles (viral vectors) can be achieved. When transfecting a nucleic acid into a cell, the composition of the present invention may be added simultaneously with the cell and the DNA encoding at least one desired component, or may be added after incubation (culture) of a cell suspension containing cells and DNA encoding at least one desired component. When the composition of the present invention is added later, the cell suspension is preferably cultured for 1 sec to 48 hr, more preferably 1 to 24 hr, and further preferably 2 to 6 hr. The culture period of the transfected cells is not particularly limited, and is, for example, 12 to 150 hr, preferably 48 to 120 hr, after addition of the composition of the present invention. The medium used for culture may contain components necessary for culturing the cells. For example, basic synthetic medium such as DMEM, IMDM, DMEM:F-12 and the like, and these basal synthetic media added as necessary with fetal bovine serum, growth factors, peptides, or increased amount of amino acids can be mentioned.

### 7. Production of antibody

In the present invention, antibodies can be produced by methods generally practiced in this technical field or methods analogous thereto. Specifically, the following steps can be performed.

A nucleic acid encoding the desired antibody is constructed based on the amino acid sequence information of the heavy chain variable region and light chain variable region of the desired antibody, and inserted into an appropriate expression vector. In addition to the nucleic acid encoding the antibody, the expression vector optionally contains a Kozak sequence to increase translation efficiency, a signal sequence to promote secretion of the antibody into the culture medium when introduced into the host, a promoter sequence, and the like. Vectors that can be used in the present invention can be selected from those generally used in this technical field, and the plasmid vector pcDNA3.4 is preferred. A plasmid vector using a promoter sequence (e.g., CHEF1) that promotes transcription of a heterologous gene can also be preferably used. Introduction of the expression vector into host cells is also not particularly limited. As methods for introducing expression vectors into host cells, the methods conventionally used in this technical field to introduce genes into cells, for example, methods known to those skilled in the art such as calcium phosphate method, electroporation method, lipofection method, and DEAE-dextran method can be used. An introduction method using lipofection is particularly preferred. As the host cells used for this purpose, those conventionally used in this technical field can also be used.

A nucleic acid encoding an antibody is inserted into an expression vector, the nucleic acid is introduced into a host cell by using the expression vector containing the nucleic acid, the host cells into which the nucleic acid has been introduced are cultured, and the antibody can be obtained from the culture supernatant by purification means such as chromatography and the like. In this method, desired antibody is secreted into the culture supernatant by culturing host cells. The antibody or antigen-binding fragments thereof can be obtained from the culture supernatant by using purification means such as chromatography and the like. As the chromatography means, various means known in this technical field such as affinity chromatography, ion exchange chromatography, and size-exclusion chromatography, and the like can be used. Affinity chromatography using a protein A column is particularly preferred.

### 8. Production of viral vector

The viral vector produced in the transfected cells can be recovered from the cells by a method generally practiced in the art. For example, extraction from viral vector-producing cells can be mentioned. Examples of the extraction method include freeze-thaw method, ultrasonic crushing method, solution extraction method, and chemical methods for appropriately adjusting the pH and salt concentration of the solution. Examples of the solution extraction method include a method of contacting producing cells with a surfactant. The method of contacting producing cells with a surfactant is performed by suspending the producing cells collected by centrifugation or filtration in a surfactant, or by adding the surfactant to a culture medium containing the producing cells. The amount of the surfactant added during extraction may vary depending on the kind of the surfactant used, and is generally 0.01 to 2.5%, preferably 0.05 to 2%, more preferably 0.1 to 1.5%, as final concentration based on the extraction solution containing the producing cells. When the amount of surfactant added is too small, a sufficient amount of viral vector is not obtained, and when it is too large, the viral vector is inactivated. When two or more kinds of surfactants are used in combination, the total amount is adjusted as appropriate to fall within the above-mentioned ranges.

Generally, a surfactant used for extraction is at least one kind selected from the group consisting of nonionic surfactant, anionic surfactant, cationic surfactant, and zwitterionic surfactant, preferably one kindselected from the group. It is more preferably a nonionic surfactant or a zwitterionic surfactant.

Nonionic surfactants refer to those that do not form ions in water. As the nonionic surfactant, known nonionic surfactants can be used. It is classified into ester type, ether type, ester ether type, alkanolamide type, sugar type, and methylglucamine type. Examples of the aforementioned ester type include polyoxyethylene sorbitan monolaurate (Tween (registered trademark) 20), polyoxyethylene sorbitan monopalmitate (Tween (registered trademark) 40), polyoxyethylene sorbitan monostearate (Tween (registered trademark) 60), polyoxyethylene sorbitan monooleate (Tween (registered trademark) 80), and the like. Examples of the ether type include polyoxyethylene alkyl ether (BriJ (registered trademark) L23, BriJ (registered trademark) L58), and polyoxyethylene isooctyl phenyl ether (Triton-X 100 (registered trademark)). Examples of the ester ether type include polyoxyethylene sorbitan fatty acid ester, polyoxyethylene hexitane fatty acid ester, sorbitan fatty acid ester polyethylene glycol, and polyoxyethylene-polyoxypropylene block copolymer (Pluronic (registered trademark)). Examples of the alkanol amide type include N,N-bis [3-(D-gluconamido)propyl]deoxycholamide. Examples of the sugar type include alkylmaltoside and alkylglucopyranoside. Examples of the methylglucamine type include alkyl-N-methylglucamine.

Anionic surfactants refer to those that ionize in water and become organic anions. As the anionic surfactant, known anionic surfactants can be used. It is classified into carboxylic acid type, sulfonic acid type, sulfuric acid ester type, and phosphoric acid ester type. Examples of those classified into the carboxylic acid type include deoxycholate, cholate, and lauroylsarcosine salt. Examples of the sulfonic acid type include dodecylsulfate. As the aforementioned salts, sodium salt and lithium salt are preferred.

Cationic surfactants refer to those that ionize in water and become organic cations. As the cationic surfactant, known cationic surfactants can be used. It is classified into alkylamine salt type and quaternary ammonium salt type. Examples of those classified into the alkylamine salt type include monomethylamine hydrochloride, dimethylamine hydrochloride, and trimethylamine hydrochloride. Examples of the quaternary ammonium salt type include hexadecyltrimethylammonium bromide, myristyltrimethylammonium bromide, and the like.

Zwitterionic surfactants refer to those that have both an anion group and a cation group in a molecule. As the zwitterionic cationic surfactant, known zwitterionic surfactants can be used. Examples include alkylamino fatty acid salt, sulfobetaine, alkylbetaine, alkylaminoxide, and the like. It is preferably alkylbetaine. As alkylbetaine, 3-[(3-cholamidopropyl)dimethyl-ammonio]propane sulfonate (CHAPS) is recited.

Here, a sufficient amount of viral vectors can be extracted by adding components other than the surfactant and necessary for extracting viral vectors. The components include endonuclease and a small amount of MgCl₂ required for activation thereof. Endonuclease is used to degrade contaminating DNA/RNA, i.e., nucleic acids derived from cells mostly containing viral vectors.

When a viral vector is extracted from a cell containing a viral vector, incubation is generally performed at 25 to 40°C, preferably 30 to 37°C, for 0.5 to 5 hr, preferably 1 to 3 hr, more preferably about 2 hr.

Viral vectors extracted from producing cells can be subjected to rough purification such as ultrafiltration, and then further purified.

Further purification includes purification by column chromatography. Purification techniques using column chromatography are well known to those skilled in the art (e.g., JP 2014-237661 A). One or more of various chromatography methods can be used for this purification. Examples of the chromatography method include affinity chromatography, ion exchange chromatography, hydrophobic chromatography, gel filtration chromatography, reversed-phase chromatography, immobilized metal ion affinity chromatography, and the like, preferably affinity chromatography, ion exchange chromatography, hydrophobic chromatography, and gel filtration chromatography.

The present invention is described in detail below using examples, but the present invention is not limited in any way. Reagents and materials to be used are commercially available unless otherwise specified, or can be prepared from known literature and the like. In addition, those skilled in the art understand that other substances having similar effects and actions can be used alternatively.

### [Example]

### Example 1

### 1. Production of viral vector by human-derived cell

In order to improve the productivity and quality of adeno-associated virus (AAV) vectors by human-derived cells, the addition of the composition of the present invention during production of AAV2 vector was investigated. Specific steps are shown below.

Suspended HEK293 cells were cultured with shaking for 3 to 4 days (37°C, 8% CO₂) in an Erlenmeyer flask containing a complete synthetic medium for suspended cells (GMEP, HE400AZ), and then the cells were grown to a cell concentration of 2.5 to 4.0×10⁶ Viable Cells/mL.

The obtained cell culture solution was collected aseptically, and cell pellets were collected by centrifugation. The cell pellets were resuspended in fresh complete synthetic medium to a cell concentration suitable for AAV2 production, transferred to an Erlenmeyer flask, and cultured with shaking (37°C, 8% CO₂).

After 3 to 4 hr, the following plasmids (i) to (iii) for production of AAV2 that expresses GFP (Green Fluorescent Protein) were added together with transfection reagents (Polyplus, FectoVIR-AAV) to the culture medium to transfect the cells.
(i) plasmid encoding Rep protein and Cap protein of AAV2 (Takara Bio Inc., pRC2-mi342 Vector)
(ii) plasmid containing E2A sequence, VA sequence, and E4 sequence of adenovirus (Takara Bio Inc., pHelper Vector)
(iii) plasmid containing expression cassette of fluorescent protein GFP between two ITRs of AAV2 (CELL BIOLABS, pAAV-GFP)

About 4 hr after transfection, filtered ethanol was added at 0.09 to 2.2 V/V% of the culture solution amounts and AAV2 was produced for 3 days.

### 2. Viral vector extraction

Three days after transfection, the culture medium of HEK293 cell that produced AAV2 was collected, the culture supernatant and cell pellet were separated by centrifugation, and the culture supernatant was frozen at -80°C. AAV2 was extracted from the cell pellet by using a commercially available AAV extraction kit (Takara, AAVpro Extraction Solution) and according to the instructions of the kit, and the lysate was frozen at -80°C.

### 3. Viral vector quantification

AAV2 extracted from the culture supernatant and from the producing cells was quantified by Droplet Digital PCR (ddPCR) method according to the procedure shown below.

DNase was added to the AAV2 extract from the culture supernatant and the producing cells to digest the nucleic acids present outside the AAV2 particles in the measurement sample, and then the activity of DNase was discontinued. Subsequently, the capsid protein of AAV2 was degraded with Proteinase K to extract the AAV genome embedded in the AAV2 particles, and then the action of Proteinase K was discontinued to obtain an AAV2 genome extract.

The obtained AAV2 genome extract was subjected to ddPCR using two kinds of primers that amplify the base sequences of the ITR (Inverted Terminal Repeat) region and GFP region of the AAV2 genome, and the amount of AAV2 produced in the sample was absolutely quantified.

In addition, from the proportion of ddPCR droplets that detected both the ITR region and the GFP region, the proportion of AAV2 containing the integrated AAV2 genome contained in the AAV2 particles containing the AAV2 genome produced under each condition was calculated.

The relative concentrations of AAV2 in cell lysates and culture supernatants when ethanol was added at various concentrations, compared to when ethanol was not added during AAV2 production are shown in Fig. 1A and Fig. 1B. The percentage of AAV2 containing the integrated genome among the AAV2s contained in the cell lysate and the culture supernatant at each addition concentration of ethanol is shown in Fig. 2A and Fig. 2B.

From the results of Fig. 1A and Fig. 1B, the addition of ethanol during AAV2 production (particularly at not exceeding 1.1 v/v%) improved the genome titer of AAV2 both in cells and in the culture supernatant. This is considered to be due to the addition of ethanol which increased the activity related to AAV2 production by AAV2-producing cells and suppressed the metabolic system that suppresses AAV2 production. In addition, within the range of the addition amount of ethanol where the AAV2 genome titer is higher in the cells than in the control, the cells proliferated at a rate equivalent to or higher than in the control condition even during AAV2 production, and the viability of the producing cells on day 3 after transfection was maintained higher than in the control condition (Fig. 1C).

From the results of Fig. 2A and Fig. 2B, the addition of ethanol during AAV2 production improved the proportion of the integrated genome in AAV2, and in particular, the percentage of integrated genome in AAV2 in the producing cells was clearly higher when ethanol was added within the range of about 2v/v%.

### 4. Quantification of the number of viral vector-derived particles

AAV2 particles (whole particles with and without AAV2 genome) extracted from the culture supernatant and producing cells were measured using a commercially available AAV2 ELISA kit (PROGENE, AAV2 Titration ELISA) and according to the instructions of the kit.

The proportion (Full rate) of AAV2 particles containing the integrated AAV2 genome was calculated from the total number of AAV2 particles quantified by the ELISA method and the quantitative value of particles containing the integrated AAV2 genome obtained from ddPCR.

The Full rate when ethanol was added and when ethanol was not added during AAV2 production is shown in Table 3.

**[Table 3]**

| sample | ethanol concentration (v/v%) | genome titer (vg/µL) | AAV2 particle number (particles/mL) | Full rate (%) |
|---|---|---|---|---|
| test (1) control | 0 | 2.26E+07 | 9.86E+11 | 2.30 |
| test (1) ethanol addition | 0.56 | 5.05E+07 | 1.41E+12 | 3.58 |
| test (2) control | 0 | 3.23E+07 | 1.04E+12 | 3.12 |
| test (2) ethanol addition | 0.56 | 3.81E+07 | 1.13E+12 | 3.38 |

| | | | | |
|---|---|---|---|---|
| *: AAV2 was all produced in cells | | | | |

From the ELISA (total AAV2 particle number) quantitative value of AAV2 produced within the cells and the results of genome titer of the same sample in the AAV2 production test shown in Table 3, it can be seen that AAV2 produced with addition of ethanol showed higher rate of incorporation of AAV2-derived genome into the produced AAV2 capsid.

### 5. Confirmation of cell infectivity of viral vector

AAV2 was extracted from the culture supernatant and producing cells and an AAV2 solution was prepared. Three days after adding the AAV2 solution to cultured HeLa cells, the number of cells expressing the GFP protein derived from the added AAV2 was measured, and the number of cells infected by the AAV2 solution per mL was calculated. The results of genome titer, genome integrity, and infection titer when ethanol was not added (control) and when ethanol was added during transfection are shown in Table 4.

**[Table 4]**

| sample | genome titer (vg/µL) | genome integrity (%) | infection titer (ifu/mL) |
|---|---|---|---|
| control condition intracellular AAV2 extract | 4.40E+07 | 45.6 | 1.36E+08 |
| ethanol addition (0.56 v/v%) condition intracellular AAV2 extract | 6.59E+07 | 54.7 | 1.63E+08 |

| | | | |
|---|---|---|---|
| *: Genome titer is ddPCR quantification value using primer that amplifies ITR region. Based on this quantitative value, cells were infected with the same amount of genome titer of intracellular AAV2 extract and the infection titer was measured. | | | |

From the results shown in Table 4, the AAV2 vector produced under condition with ethanol addition and showing improved viral genome integrity had a higher value of infecting cells and expressing GFP per volume of lysate from the cells, and showed a high infection titer.

### 6. Proteome analysis and pathway analysis during viral vector production

In order to identify metabolic changes that occurred in AAV2-producing cells when ethanol was added and important factors, intracellular proteins were extracted from cells with and without addition of ethanol during AAV2 production, the extracted proteins were analyzed by liquid chromatography-mass spectrometry (LC-MS) and proteins whose expression increased or decreased during AAV2 production due to the presence or absence of ethanol addition were analyzed. Based on the obtained proteome analysis results, and using a pathway analysis software, Ingenuity Pathway Analysis (IPA, Qiagen), pathways activated or suppressed during AAV2 production due to the presence or absence of ethanol addition, and the proteins that affected pathway control were identified.

A heat map of the pathways that changed during AAV2 production by the presence or absence of ethanol addition is shown in Fig. 3. The cell functions affected by the changed pathways and, among the proteins related to the control of each pathway, proteins that were particularly increased or decreased when the pathways changed significantly are shown in Table 5 and Table 6.

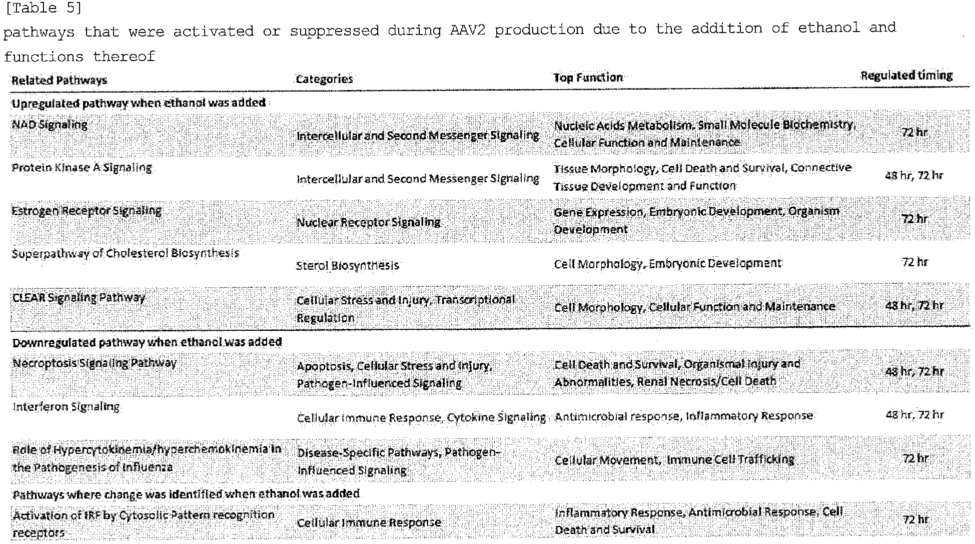

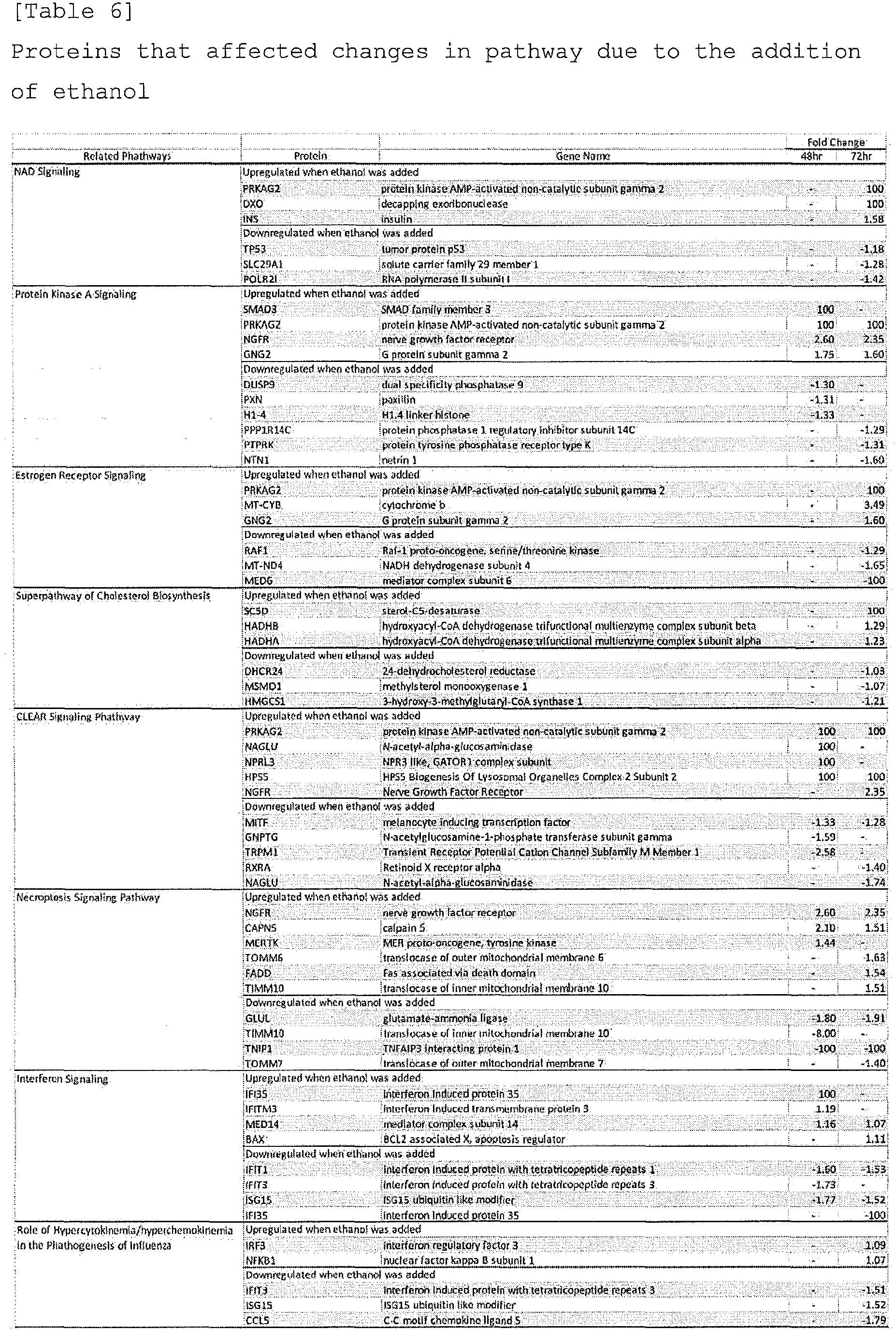

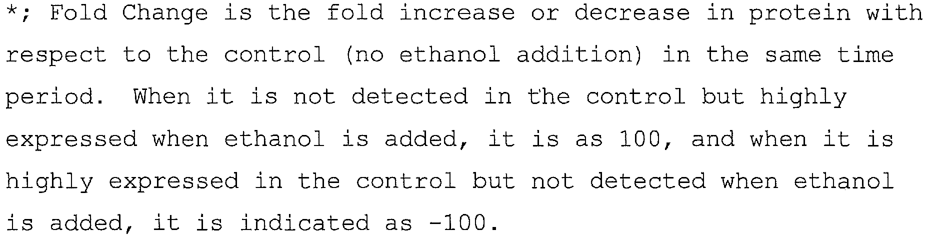

From the results shown in Fig. 3 and Table 5, the addition of ethanol activated, at 48 and 72 hours after transfection of plasmid DNA for AAV2 production, NAD Signaling and Protein Kinase A Signaling, which are involved in intracellular signal transduction, Estrogen Receptor Signaling, which is involved in the control of gene expression, Superpathway of Cholesterol Biosynthesis, which is involved in cell morphology and intracellular maintenance, and CLEAR Signaling Pathway. On the other hand, pathways relating to cell death and immunity against viral infection such as Necroptosis Signaling Pathway, Interferon Signaling, Role of Hypercytokinemia/hyperchemokinemia in the Pathogenesis of influenza, and the like were suppressed. Changes of these pathways support that the addition of ethanol has the effect of improving AAV productivity, and cell proliferation and survival rate even during AAV production.

Furthermore, the proteins in Table 6 showed the expression levels significantly increased or decreased due to the addition of ethanol, among the proteins involved in the control of respective pathways, and the increased expression of proteins such as PRKAG2, NGFR, and GNG2 is significantly involved in the activation of cells. In particular, SMAD3 is a protein involved in the early stages of pathway in Protein Kinase A Signaling, and an increase thereof greatly contributes to the activation of pathways.

In addition, decreased expression of proteins such as GLUL, TIMM10, TNIP1, TOMM7, IFIT1, IFIT3, ISG15, and IFI35 significantly contributes to the suppression of pathways, such as Necroptosis Signaling and Interferon Signaling, related to cell death and resistance to viruses such as AAV.

### Example 2

In the same manner as in Example 1, "1. Production of viral vector by human-derived cell" except that the composition of the present invention to be added was changed from ethanol to glycerol, methanol, and isopropanol, production of viral vector and analysis were performed, and the amount of AAV2 produced and the integrity of the genome in the produced AAV2 particles 3 days after transfection in the producing cells were measured. The measurement results are shown in Table 7.

**[Table 7]**

| sample | additive concentration (v/v%) | genome titer (vg/pL) | genome integrity (%) |
|---|---|---|---|
| test (3) control | 0.00 | 2.26E+07 | 51.9 |
| test (3) Glycerol addition | 0.73 | 4.50E+07 | 58.9 |
| test (4) control | 0.00 | 2.40E+07 | 53.4 |
| test (4) Glycerol addition | 0.38 | 2.78E+07 | 55.2 |
| test (4) Glycerol addition | 1.15 | 3.66E+07 | 58.5 |
| test (4) Methanol addition | 0.33 | 2.94E+07 | 58.4 |
| test (4) Isopropanol addition | 0.61 | 2.26E+07 | 58.5 |

| | | | |
|---|---|---|---|
| *: AAV2 was all produced in cells | | | |

From the results shown in Table 7, it can be seen that even alcohols other than ethanol are effective in improving the integrity of the genome in the produced AAV2 and improving the genome titer (productivity), and that there is an appropriate concentration range for the effect, similar to when ethanol is added.

### Example 3

In the same manner as in Example 1, "1. Production of viral vector by human-derived cell" except that the composition of the present invention to be added was changed from ethanol to dimethyl sulfoxide (DMSO), production of viral vector and analysis were performed. The results relating to the culture supernatant and the producibility of AAV2 in the cells 3 days after transfection are shown in Table 8.

**[Table 8]**

| sample | additive concentration (v/v%) | genome titer (vg/µL) | genome integrity (%) | TFday3 VCD (cells/mL) | TF day3 Viability (%) |
|---|---|---|---|---|---|
| test (5) control | 0.00 | 2.40E+07 | 53.4 | 4.17E+06 | 78.5 |
| test (5) DMSO addition | 0.57 | 3.35E+07 | 59.3 | 4.58E+06 | 81.5 |

| | | | | | |
|---|---|---|---|---|---|
| *: values relating to AAV2 produced in cells | | | | | |

From the results shown in Table 8, it can be seen that even DMSO which is other than alcohols is effective in improving the integrity of the genome in the produced AAV2 and improving the genome titer (productivity). Also, it can be seen that even though DMSO is generally said to have a protective function during cryopreservation of cells and also said to be toxic to cells, it has the effect of improving cell growth and substance production when the concentration is low.

### Example 4

In the same manner as in Example 1, "1. Production of viral vector by human-derived cell" except that, among the transfected three types of plasmid DNAs, (i) plasmid encoding Rep protein and Cap protein of AAV2 was changed to (iv) plasmid encoding Rep protein and Cap protein of AAV1 or (v) plasmid encoding Rep protein and Cap protein of AAV6, production of viral vector and analysis were performed. The results relating to the culture supernatant and the producibility of AAV1 and AAV6 in the cells 3 days after transfection are shown in Table 9.

**[Table 9]**

| sample | ethanol addition concentration (v/v%) | culture supernatant | | cell lysate | |
|---|---|---|---|---|---|
| | | genome titer (vg/µL) | genome integrity (%) | genome titer (vg/µL) | genome integrity (%) |
| AAV1 control | 0.00 | 3.34E+06 | 49.3 | 2.67E+06 | 43.3 |
| AAV1 ethanol addition | 0.28 | 5.09E+06 | 58.6 | 4.67E+06 | 54.2 |
| AAV6 control | 0.00 | 1.20E+05 | 23.7 | 1.60E+07 | 51.8 |
| AAV6 ethanol addition | 0.28 | 1.08E+05 | 28.6 | 2.05E+07 | 55.1 |

From the results shown in Table 9, it can be seen that, also in the production of AAV1, the amount of AAV1 produced both in the culture supernatant and within the cells (genome titer) and the genome integrity were improved when ethanol was added. Furthermore, also in the production of AAV6, the amount of AAV6 produced within cells (genome titer) was improved when ethanol was added, and the genome integrity was improved in AAV6 produced both in the culture supernatant and within the cells. It is found therefrom that the addition of ethanol is effective in improving productivity and the integrity of packaged genome in various serotypes of AAV.

### Example 5

### 1. Production of antibody by Chinese hamster-derived cell

In order to improve the productivity of antibody by Chinese hamster-derived cells (CHO cells), the addition of the composition of the present invention during production of antibody was investigated. Specific steps are shown below.

An expression plasmid for antibody production was introduced into suspended CHO cells (DG44) and the cells were cultured with shaking for 2 to 3 days (37°C, 5% CO₂) in an Erlenmeyer flask containing a commercially available medium for suspended cells, and then the cells were grown to a cell concentration of 1.0 to 4.0×10⁶ Viable Cells/mL.

The obtained cell culture solution was collected aseptically, and cell pellets were collected by centrifugation. The cell pellets were resuspended in a fresh medium to a cell concentration of 5.0×10⁵ Viable Cells/mL suitable for antibody production, and transferred to an Erlenmeyer flask (37°C, 5% CO₂).

Thereafter, filtered ethanol was at 0.25 V/V%, 0.5 V/V%, and 0.75 V/V% of the culture solution amounts and the antibody (Bevacizumab) was produced for 7 days. Ethanol was not added to the control (0 V/V%).

### 2. Recovery of culture supernatant

After 7 days from ethanol addition, the culture medium (1 mL) was collected in a tube, centrifuged at 9730×g, 1 min, 4°C, and the supernatant (0.5 mL) was newly separated and stored at -80°C.

### 3. Antibody quantification

Samples stored at -80°C were thawed, and the concentration of antibody contained in the culture supernatant was quantified using Cedex Bio HT and according to the use manual of IgG Bio HT kit (manufactured by Roche Diagnostics). The results are shown in Fig. 4.

### [Industrial Applicability]

According to the method of the present invention, it is possible to produce antibody more superior in the production amount of antibody, and to produce viral vectors more superior in the production amount and/or integrity of viral genome.

This application is based on a patent application No. 2021-178303 filed in Japan (filing date: October 29, 2021) and a patent application No. 2022-072677 filed in Japan (filing date: April 26, 2022), the contents of which are incorporated in full herein.

## Claims

1. A composition for addition to cells, which comprises an organic solvent, wherein the organic solvent enhances in the cell the production of at least one desired component.

2. The composition according to claim 1, wherein a signal related to cell survival has been regulated in the cell after the addition compared to the cell before the addition.

3. The composition according to claim 1, which increases survival of the cell and/or activity of the cell.

4. The composition according to claim 1, which is for the transfection of a nucleic acid.

5. The composition according to claim 1, which is for the enhanced expression of a nucleic acid.

6. The composition according to claim 4 or 5, wherein the nucleic acid is a DNA encoding a protein.

7. The composition according to claim 6, wherein the aforementioned DNA is a DNA involved in the production of at least one kind selected from the group consisting of an antibody and a viral vector.

8. The composition according to claim 1, wherein the organic solvent comprises an alcohol and/or dimethyl sulfoxide.

9. A method for enhancing the production of at least one desired component in a cell, comprising contacting the cell with a composition comprising an organic solvent.

10. A method for enhancing the production of at least one desired component in a cell, comprising incubating a reaction mixture comprising the cell and an organic solvent.

11. The method according to claim 9 or 10, wherein the aforementioned cell is a cell into which a DNA involved in the production of at least one desired component has been introduced.

12. The method according to claim 9 or 10, wherein a signal related to cell survival has been regulated in the cell after contact with the composition comprising the organic solvent, or after incubation, compared to the cell before the contact or incubation.

13. The method according to claim 9 or 10, which increases survival of the cell and/or activity of the cell.

14. A method for transfecting a nucleic acid encoding a desired component into a cell, comprising incubating a reaction mixture comprising a nucleic acid encoding at least one desired component, a cell, and an organic solvent.

15. The method according to claim 14, which enhances the expression of the transfected nucleic acid.

16. The method according to claim 14, wherein a signal related to cell survival has been regulated in the cell after the transfection compared to the cell before the transfection.

17. The method according to claim 14, wherein the nucleic acid is a DNA encoding a protein.

18. The method according to claim 17, wherein the aforementioned DNA is a DNA involved in the production of at least one kind selected from the group consisting of an antibody and a viral vector.

19. The method according to claim 14, wherein the organic solvent comprises an alcohol and/or dimethyl sulfoxide.

20. The method according to claim 14, wherein the reaction mixture further comprises a transfection reagent.

21. A method for transfecting a DNA encoding a desired component into a cell, comprising
a first step of incubating a cell suspension comprising a cell and a DNA encoding at least one desired component,
a second step of preparing a reaction mixture by adding an organic solvent to the incubated cell suspension, and
a third step of incubating the reaction mixture, wherein
the organic solvent is an alcohol.

22. A method for producing a transfected cell, comprising transfecting a cell by the method according to claim 14.

23. A method for producing a viral vector, comprising obtaining a viral vector from a cell obtained by the production method according to claim 22.
